# EUROPEAN PATENT APPLICATION

(11) **EP 4 598 008 A1**
(43) Date of publication of application: **06.08.2025**
(21) Application number: 22963281.5
(22) Date of filing: 28.11.2022
(51) Int. Cl.: H04N 7/14, A61B 5/0205

(54) **MONITORING INFORMATION DISPLAY METHOD, MONITORING SYSTEM, AND RELATED DEVICE**

(30) Priority: 28.10.2022 WO PCT/CN2022/128414
(71) Applicant: Shenzhen Mindray Bio-Medical Electronics Co., Ltd., Shenzhen, Guangdong 518057 (CN)
(72) Inventor: LI, Liya, Shenzhen, Guangdong 518057 (CN); WU, Jiao, Shenzhen, Guangdong 518057 (CN); QING, Lei, Shenzhen, Guangdong 518057 (CN); LIU, Zhonghua, Shenzhen, Guangdong 518057 (CN); YUAN, Weiwei, Shenzhen, Guangdong 518057 (CN)
(74) Representative: KIPA AB
(86) International application number: PCT/CN2022/134639
(87) International publication number: WO 2024/087302

(57) **Abstract**

Embodiments of the present application provide a monitoring information display method, a monitoring system, a bedside monitoring device, and a mobile monitoring device. The monitoring system comprises: the bedside monitoring device and the mobile monitoring device that can be attached to a human body; when a display mode of the bedside monitoring device is a first display mode and the mobile monitoring device works in the first monitoring mode, the bedside monitoring device acquires first physiological parameter information and wearing activity information, and displays same separately; and when the display mode of the bedside monitoring device is a second display mode and the mobile monitoring device works in the second monitoring mode, the bedside monitoring device at least acquires second physiological parameter information and displays same.

## Description

### CROSS-REFERENCE OF RELATED APPLICATIONS

The application is based on an international patent application No. PCT/CN2022/128414, filed on October 28, 2022, and titled "INFORMATION DISPLAY METHOD AND BEDSIDE MONITORING DEVICE", and claims priority to said international patent application; the contents of which are incorporated herein by reference in their entirety.

### TECHNICAL FIELD

The disclosure relates to a technical field of medical device, and in particular to a display method for monitoring information, a monitoring system and related device.

### BACKGROUND

Bedside monitor can display a physiological parameter signal acquired from a human body, so as to allow medical staff to check in time and determine a physical condition of a patient.

At present, a mobile monitoring device can acquire a physiological parameter signal of a human body when a patient moves. Bedside monitor usually display, in a fixed mode, physiological parameter information determined by the physiological parameter signal acquired autonomously by a mobile monitoring device or a bedside monitor. Accordingly, its monitoring display effect is single.

### SUMMARY

Embodiments of this disclosure are intended to provide a display method for monitoring information, a monitoring system and related device.

A technical solution of an embodiment of this disclosure is implemented as follows.

An embodiment of this disclosure provides a monitoring system, including: a bedside monitoring device, and a mobile monitoring device which is capable of being worn by a human body;
a display mode of the bedside monitoring device includes a first display mode and a second display mode;
a monitoring mode of the mobile monitoring device includes a first monitoring mode and a second monitoring mode;
a frequency of the mobile monitoring device when operating in the first monitoring mode is lower than a frequency of the mobile monitoring device when operating in the second monitoring mode; wherein a frequency of the mobile monitoring device when operating in the first monitoring mode being lower than a frequency of the mobile monitoring device when operating in the second monitoring mode, includes at least one of following two situations: a signal acquisition frequency of the mobile monitoring device when operating in the first monitoring mode is lower than a signal acquisition frequency of the mobile monitoring device when operating in the second monitoring mode, and a data transmission frequency of the mobile monitoring device when operating in the first monitoring mode is lower than a data transmission frequency of the mobile monitoring device when operating in the second monitoring mode;
when the display mode of the bedside monitoring device is the first display mode and the mobile monitoring device operates in the first monitoring mode, the bedside monitoring device is configured to obtain first physiological parameter information and wear activity information, and distinguishingly display the first physiological parameter information and the wear activity information;
when the display mode of the bedside monitoring device is the second display mode and the mobile monitoring device operates in the second monitoring mode, the bedside monitoring device is configured to at least obtain second physiological parameter information, and display the second physiological parameter information;
the first physiological parameter information and the second physiological parameter information are obtained by the bedside monitoring device through receiving and processing a physiological parameter signal of the human body acquired by the mobile monitoring device, or by the bedside monitoring device through receiving physiological parameter information of the human body generated by the mobile monitoring device;
the wear activity information is obtained by the bedside monitoring device through receiving and at least processing a non-physiological parameter signal acquired by the mobile monitoring device, or by the bedside monitoring device through receiving the wear activity information of the human body generated by the mobile monitoring device;
wherein the wear activity information includes at least one of: posture information, position information, movement time, and sleep time, of the human body.

An embodiment of this disclosure provides a display method for monitoring information, which method is applied to the monitoring system, wherein the monitoring system includes a bedside monitoring device, and a mobile monitoring device which is capable of being worn by a human body; a display mode of the bedside monitoring device includes a first display mode and a second display mode; a monitoring mode of the mobile monitoring device includes a first monitoring mode and a second monitoring mode;
wherein the method includes:
when the display mode of the bedside monitoring device is the first display mode and the mobile monitoring device operates in the first monitoring mode:
receiving, by the bedside monitoring device, a physiological parameter signal of the human body acquired by the mobile monitoring device, and processing, by the bedside monitoring device, the physiological parameter signal of the human body to obtain first physiological parameter information; or receiving, by the bedside monitoring device, first physiological parameter information of the human body generated by the mobile monitoring device;
receiving, by the bedside monitoring device, a non-physiological parameter signal acquired by the mobile monitoring device, and at least processing, by the bedside monitoring device, the non-physiological parameter signal to obtain wear activity information; or receiving, by the bedside monitoring device, wear activity information of the human body generated by the mobile monitoring device, wherein the wear activity information includes at least one of: posture information, position information, movement time, and sleep time, of the human body;
distinguishingly displaying, by the bedside monitoring device, the first physiological parameter information and the wear activity information;
when the display mode of the bedside monitoring device is the second display mode and the mobile monitoring device operates in the second monitoring mode:
   receiving, by the bedside monitoring device, a physiological parameter signal of the human body acquired by the mobile monitoring device, and processing, by the bedside monitoring device, the physiological parameter signal of the human body to obtain second physiological parameter information; or receiving, by the bedside monitoring device, second physiological parameter information of the human body generated by the mobile monitoring device;
   displaying, by the bedside monitoring device, the second physiological parameter information.

An embodiment of this disclosure provides a bedside monitoring device, including a communication unit, a processor, a memory and a display;
the communication unit is in wireless communication with a mobile monitoring device which is capable of being worn by a human body;
the communication unit is configured to receive a physiological parameter signal of the human body acquired by the mobile monitoring device, and the processor is configured to process the physiological parameter signal of the human body, so as to obtain physiological parameter information, or the communication unit is configured to receive physiological parameter information of the human body generated by the mobile monitoring device;
the communication unit is further configured to receive a non-physiological parameter signal acquired by the mobile monitoring device, and the processor is further configured to at least process the non-physiological parameter signal, so as to obtain wear activity information, or the communication unit is further configured to receive wear activity information of the human body generated by the mobile monitoring device;
the memory is configured to store executable instructions, and the processor is further configured to execute the executable instructions, so as to enable the processor to perform following operations:
   when a display mode of the bedside monitoring device is a first display mode, obtaining first physiological parameter information and the wear activity information, and controlling the display to distinguishingly display the first physiological parameter information and the wear activity information;
   when a display mode of the bedside monitoring device is a second display mode, obtaining at least second physiological parameter information, and controlling the display to display the second physiological parameter information;
   wherein the wear activity information includes at least one of: posture information, position information, movement time, and sleep time, of the human body.

An embodiment of this disclosure provides a mobile monitoring device, wherein a monitoring mode of the mobile monitoring device includes a first monitoring mode and a second monitoring mode;
a frequency of the mobile monitoring device when operating in the first monitoring mode is lower than a frequency of the mobile monitoring device when operating in the second monitoring mode; wherein a frequency of the mobile monitoring device when operating in the first monitoring mode being lower than a frequency of the mobile monitoring device when operating in the second monitoring mode, includes at least one of following two situations: a signal acquisition frequency of the mobile monitoring device when operating in the first monitoring mode is lower than a signal acquisition frequency of the mobile monitoring device when operating in the second monitoring mode, and a data transmission frequency of the mobile monitoring device when operating in the first monitoring mode is lower than a data transmission frequency of the mobile monitoring device when operating in the second monitoring mode;
wherein the mobile monitoring device includes a first acquisition module, a second acquisition module, a processor and a communication unit, wherein:
   the first acquisition module is configured to acquire a physiological parameter signal of a human body, or process a physiological parameter signal acquired to obtain physiological parameter information of the human body;
   the second acquisition module is configured to acquire a non-physiological parameter signal, or at least process a non-physiological parameter signal acquired to obtain wear activity information of the human body;
   the communication unit is configured to establish a communicative connection with a bedside monitoring device;
   the processor is configured to perform following operations:
      when the monitoring mode of the mobile monitoring device is the first monitoring mode, transmitting to the bedside monitoring device, through the communication unit, a first physiological parameter signal or first physiological parameter information, and the non-physiological parameter signal or the wear activity information, so as to enable the bedside monitoring device to display the first physiological parameter information and the wear activity information; wherein the wear activity information includes at least one of: posture information, position information, movement time, and sleep time, of the human body;
      when the monitoring mode of the mobile monitoring device is the second monitoring mode, transmitting to the bedside monitoring device, through the communication unit, a second physiological parameter signal or second physiological parameter information, so as to enable the bedside monitoring device to display the second physiological parameter information.

An embodiment of this disclosure provides a display method for monitoring information, which is applied to a bedside monitoring device, wherein the bedside monitoring device is in communicative connection with a mobile monitoring device which is capable of being worn by a human body;
the method includes:
receiving, by the bedside monitoring device, a physiological parameter signal of the human body acquired by the mobile monitoring device, and processing, by the bedside monitoring device, the physiological parameter signal of the human body to obtain first physiological parameter information; or receiving, by the bedside monitoring device, first physiological parameter information of the human body generated by the mobile monitoring device;
receiving, by the bedside monitoring device, a non-physiological parameter signal acquired by the mobile monitoring device, and at least processing, by the bedside monitoring device, the non-physiological parameter signal to obtain wear activity information; or receiving, by the bedside monitoring device, wear activity information of the human body generated by the mobile monitoring device;
distinguishingly displaying, by the bedside monitoring device, the first physiological parameter information and the wear activity information;
wherein the wear activity information includes at least one of: posture information, position information, movement time, and sleep time, of the human body.

An embodiment of this disclosure provides a bedside monitoring device, including a communication unit, a processor, a memory and a display;
the communication unit is in wireless communication with a mobile monitoring device which is capable of being worn by a human body;
the communication unit is configured to receive a physiological parameter signal of the human body acquired by the mobile monitoring device, and the processor is configured to process the physiological parameter signal of the human body, so as to obtain physiological parameter information, or the communication unit is configured to receive physiological parameter information of the human body generated by the mobile monitoring device;
the communication unit is further configured to receive a non-physiological parameter signal acquired by the mobile monitoring device, and the processor is further configured to at least process the non-physiological parameter signal, so as to obtain wear activity information, or the communication unit is further configured to receive wear activity information of the human body generated by the mobile monitoring device;
the memory is configured to store executable instructions, and the processor is further configured to execute the executable instructions, so as to enable the processor to perform following operations:
   acquiring first physiological parameter information and the wear activity information, and controlling the display to distinguishingly display the first physiological parameter information and the wear activity information;
   wherein the wear activity information includes at least one of: posture information, position information, movement time, and sleep time, of the human body.

An embodiment of this disclosure provides a mobile monitoring device, including a first acquisition module, a second acquisition module, a processor and a communication unit, wherein:
the first acquisition module is configured to acquire a physiological parameter signal of a human body, or process a physiological parameter signal acquired to obtain physiological parameter information of the human body;
the second acquisition module is configured to acquire a non-physiological parameter signal, or at least process a non-physiological parameter signal acquired to obtain wear activity information of the human body;
the communication unit is configured to establish a communicative connection with a bedside monitoring device;
the processor is configured to perform following operations:
   transmitting to the bedside monitoring device, through the communication unit, a first physiological parameter signal or first physiological parameter information; and transmitting to the bedside monitoring device, through the communication unit, the non-physiological parameter signal or the wear activity information, so as to enable the bedside monitoring device to display the first physiological parameter information and the wear activity information;
   wherein the wear activity information includes at least one of: posture information, position information, movement time, and sleep time, of the human body.

An embodiment of this disclosure provides a monitoring system, including: a bedside monitoring device, and a mobile monitoring device which is capable of being worn by a human body;
wherein the bedside monitoring device is configured to establish a communicative connection with the mobile monitoring device;
wherein, the bedside monitoring device is the above-mentioned bedside monitoring device.

Embodiments of this disclosure provide a display method for monitoring information, a monitoring system and related device, wherein the monitoring system includes: a bedside monitoring device, and a mobile monitoring device which is capable of being worn by a human body; a display mode of the bedside monitoring device includes a first display mode and a second display mode; a monitoring mode of the mobile monitoring device includes a first monitoring mode and a second monitoring mode; a frequency of the mobile monitoring device when operating in the first monitoring mode is lower than a frequency of the mobile monitoring device when operating in the second monitoring mode; wherein a frequency of the mobile monitoring device when operating in the first monitoring mode being lower than a frequency of the mobile monitoring device when operating in the second monitoring mode, includes at least one of following two situations: a signal acquisition frequency of the mobile monitoring device when operating in the first monitoring mode is lower than a signal acquisition frequency of the mobile monitoring device when operating in the second monitoring mode, and a data transmission frequency of the mobile monitoring device when operating in the first monitoring mode is lower than a data transmission frequency of the mobile monitoring device when operating in the second monitoring mode; when the display mode of the bedside monitoring device is the first display mode and the mobile monitoring device operates in the first monitoring mode, the bedside monitoring device is configured to obtain first physiological parameter information and wear activity information, and distinguishingly display the first physiological parameter information and the wear activity information; when the display mode of the bedside monitoring device is the second display mode and the mobile monitoring device operates in the second monitoring mode, the bedside monitoring device is configured to at least obtain second physiological parameter information, and display the second physiological parameter information; the first physiological parameter information and the second physiological parameter information are obtained by the bedside monitoring device through receiving and processing a physiological parameter signal of the human body acquired by the mobile monitoring device, or by the bedside monitoring device through receiving physiological parameter information of the human body generated by the mobile monitoring device; the wear activity information is obtained by the bedside monitoring device through receiving and at least processing a non-physiological parameter signal acquired by the mobile monitoring device, or by the bedside monitoring device through receiving the wear activity information of the human body generated by the mobile monitoring device; wherein the wear activity information includes at least one of: posture information, position information, movement time, and sleep time, of the human body. Technical solutions provided in embodiments of this disclosure enables a bedside monitoring device to display monitoring information in different display modes according to different monitoring modes of a mobile monitoring device, supports diverse monitoring displays, and improves a monitoring display effect.

### BRIEF DESCRIPTION OF THE DRAWINGS

FIG. 1 is a structure diagram of a monitoring system provided in an embodiment of this disclosure.
FIG. 2 is a structure diagram of a bedside monitoring device provided in an embodiment of this disclosure.
FIG. 3 is a structure diagram of a mobile monitoring device provided in an embodiment of this disclosure.
FIG. 4 is a schematic diagram of an exemplary display interface provided in an embodiment of this disclosure.
FIG. 5 is a first schematic diagram of an exemplary area division of a display interface provided in an embodiment of this disclosure.
FIG. 6 is a second schematic diagram of an exemplary display interface provided in an embodiment of this disclosure.
FIG. 7 is a third schematic diagram of an exemplary display interface provided in an embodiment of this disclosure.
FIG. 8 is a fourth schematic diagram of an exemplary display interface provided in an embodiment of this disclosure.
FIG. 9 is a fifth schematic diagram of an exemplary display interface provided in an embodiment of this disclosure.
FIG. 10 is a sixth schematic diagram of an exemplary display interface provided in an embodiment of this disclosure.
FIG. 11 is a seventh schematic diagram of an exemplary display interface provided in an embodiment of this disclosure.
FIG. 12 is an eighth schematic diagram of an exemplary display interface provided in an embodiment of this disclosure.
FIG. 13 is a ninth schematic diagram of an exemplary display interface provided in an embodiment of this disclosure.
FIG. 14 is a tenth schematic diagram of an exemplary display interface provided in an embodiment of this disclosure.
FIG. 15 is a first flowchart of a display method for monitoring information according to an embodiment of this disclosure.
FIG. 16 is a second flow chart of a display method for monitoring information provided in an embodiment of this disclosure.
FIG. 17 is a third flow chart of a display method for monitoring information provided in an embodiment of this disclosure.

### DESCRIPTION OF THE PREFERRED EMBODIMENTS

In order to make purposes, technical solutions and advantages of embodiments of this disclosure clearer, technical solutions in embodiments of this disclosure will be clearly and completely described below in conjunction with the drawings in the embodiments of this disclosure. Obviously, the described embodiments are only part of the embodiments of this disclosure, not all of the embodiments. The following examples are used to illustrate this disclosure but are not used to limit the scope of this disclosure. Based on the embodiments in this disclosure, all other embodiments obtained by ordinary technicians in the field without making any creative work shall fall within the protection scope of this disclosure.

In the following description, reference is made to "some embodiments", which describe a subset of all possible embodiments, but it will be understood that "some embodiments" may be the same subset or different subsets of all possible embodiments and may be combined with each other without conflict.

It should be pointed out that the terms "first\second\third" involved in the embodiments of this disclosure are merely used to distinguish similar objects and do not represent a specific ordering of the objects. It can be understood that the preset order or sequence of "first\second\third" can be interchanged where permitted, so that the embodiments of this disclosure described here can be implemented in an order other than that illustrated or described here.

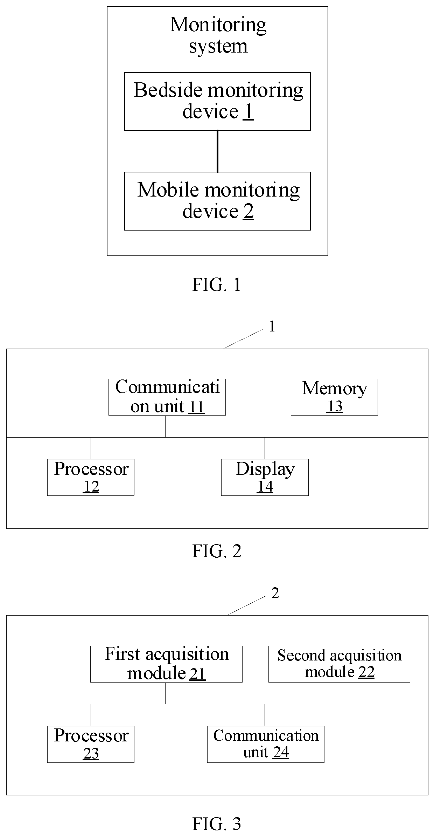

An embodiment of this disclosure provides a monitoring system. FIG. 1 is a structure diagram of a monitoring system provided in an embodiment of this disclosure. As shown in FIG. **1****,** in an embodiment of this disclosure, the monitoring system includes: a bedside monitoring device **1,** and a mobile monitoring device 2 which is capable of being worn by a human body; the bedside monitoring device 1 is configured to establish a communicative connection with the mobile monitoring device 2.

It should be noted that, in the embodiments of this disclosure, the bedside monitoring device 1 is usually placed at a fixed position, such as inside a ward, and cannot move with the human body, while the mobile monitoring device 2 can be worn by the human body and can move with the human body. Both the bedside monitoring device 1 and the mobile monitoring device 2 can perform medical monitoring on the human body. The communicative connection established between the bedside monitoring device 1 and the mobile monitoring device 2 may be a wireless communicative connection, or of course, can be a wired communicative connection.

It should be noted that in the embodiments of this disclosure, the mobile monitoring device 2 is capable of acquiring a physiological parameter signal of the human body, which signal may specifically include an electrocardiogram signal, a respiratory signal, a blood oxygen signal, a blood pressure signal, and a body temperature signal of the human body. This signal can be processed to obtain corresponding physiological parameter information of respective types, such as an electrocardiogram parameter, a respiratory parameter, a blood oxygen saturation parameter, a blood pressure parameter, and a body temperature parameter. The mobile monitoring device 2 includes at least one physiological parameter monitoring module, and the physiological parameter signal is determined via the at least one physiological parameter monitoring module.

It should be noted that, in the embodiment of this disclosure, the mobile monitoring device 2 is capable of acquiring a physiological parameter signal of the human body and a non-physiological parameter signal, when the human body is at different positions. The non-physiological parameter signal is determined by the mobile monitoring device 2 which is capable of being worn by the human body through monitoring a sensor signal of the mobile monitoring device 2 itself. For example, the mobile monitoring device 2 may also include a device, such as a gyroscope, an accelerometer, and the likes, so as to monitor the sensor signal of the mobile monitoring device 2 itself, and determine wear activity information, such as position information and posture information.

It should be noted that in the embodiment of this disclosure, the bedside monitoring device 1 can also acquire a physiological parameter signal of the human body, and type(s) of the physiological parameter signal acquired by the bedside monitoring device 1 can be at least partially same as, or completely different from type(s) of the physiological parameter signal acquired by the mobile monitoring device 2, and the embodiment of this disclosure is not limited thereto.

FIG. 2 is a structure diagram of a bedside monitoring device provided in an embodiment of this disclosure. As shown in FIG. 2, in an embodiment of this disclosure, the bedside monitoring device 1 includes: a communication unit 11, a processor 12, a memory 13, and a display 14.

The communication unit 11 is in wireless communication with the mobile monitoring device 2 which is capable of being worn by the human body.

The communication unit 11 is configured to receive a physiological parameter signal of the human body acquired by the mobile monitoring device 2 and the processor 12 is configured to process the physiological parameter signal of the human body to obtain physiological parameter information, or the communication unit 11 is configured to receive physiological parameter information of the human body generated by the mobile monitoring device 2.

The communication unit 11 is further configured to receive a non-physiological parameter signal acquired by the mobile monitoring device 2 and the processor 12 is further configured to process the non-physiological parameter signal to obtain wear activity information, or the communication unit 11 is further configured to receive wear activity information of the human body generated by the mobile monitoring device 2.

The memory 13 is configured to store executable instructions, and the processor 12 is configured to execute the executable instructions, so as to enable the processor 12 perform following operations.

The processor 12 obtains first physiological parameter information and the wear activity information, and controls the display 14 to distinguishingly display the first physiological parameter information and the wear activity information.

The wear activity information includes at least one of: posture information, position information, movement time, and sleep time, of the human body.

FIG. 3 is a structure diagram of a mobile monitoring device provided in an embodiment of this disclosure. As shown in FIG. 3, in an embodiment of this disclosure, the mobile monitoring device 2 includes a first acquisition module 21, a second acquisition module 22, a processor 23 and a communication unit 24.

The first acquisition module 21 is configured to acquire a physiological parameter signal of a human body or process physiological parameter signal acquired to obtain physiological parameter information of the human body.

The second acquisition module 22 is configured to acquire a non-physiological parameter signal or at least process a non-physiological parameter signal acquired to obtain wear activity information of the human body.

The communication unit 24 is configured to establish a communicative connection with the bedside monitoring device 1.

The processor 23 is configured to perform the following operations:
transmitting to the bedside monitoring device **1,** through the communication unit 24, a first physiological parameter signal or first physiological parameter information; and transmitting to the bedside monitoring device **1,** through the communication unit 24, the non-physiological parameter signal or the wear activity information, so as to enable the bedside monitoring device 1 to display the first physiological parameter information and the wear activity information;
wherein the wear activity information includes at least one of: posture information, position information, movement time, and sleep time, of the human body.

It should be noted that in the embodiments of this disclosure, referring to the above-mentioned bedside monitoring device 1 and mobile monitoring device 2, it can be that the bedside monitoring device 1 determines the physiological parameter information of the human body and/or the wear activity information, or that the mobile monitoring device 2 determines the physiological parameter information of the human body and/or the wear activity information. The specific execution entity can be determined according to actual requirements and application scenarios, and the embodiments of this disclosure are not limited.

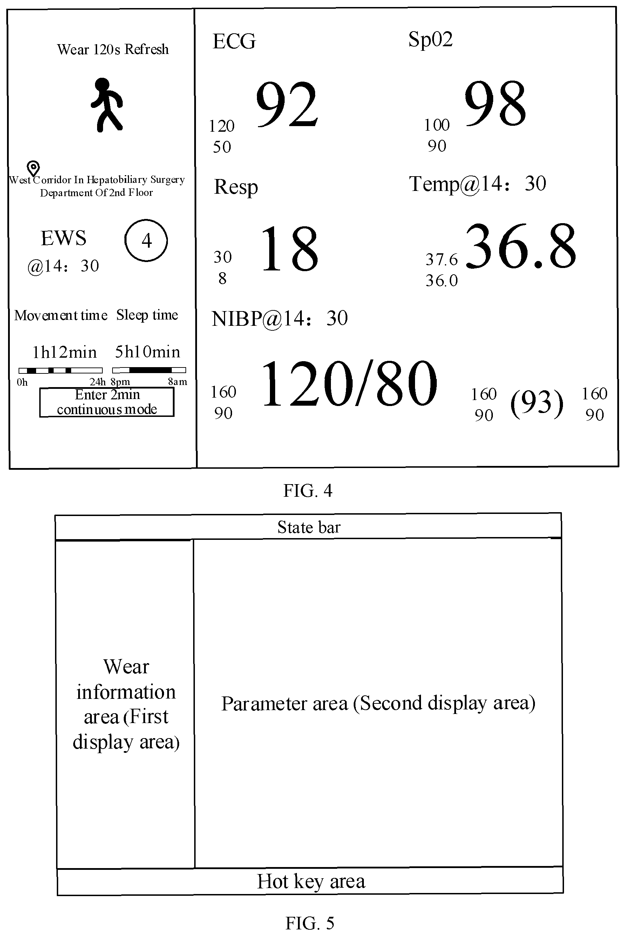

Exemplarily, in an embodiment of this disclosure, as shown in FIG. 4, the processor 12 in the bedside monitoring device 1 controls the display 14 to display the first physiological parameter information, which includes an electrocardiogram (ECG) parameter, a respiration (Resp) parameter, a blood oxygen saturation (SpO2) parameter, a blood pressure (NIBP) parameter, and a body temperature (Temp) parameter, wherein these physiological parameter are displayed only in a numerical form. The wear activity information includes posture information, position information, movement time, sleep time, and EWS score, of the human body. As shown in FIG. 4, in the wear activity information, the posture information is displayed in a form of an icon which represents a posture of human body, specifically an icon of a walking person. The position information is displayed in a form of text, specifically "West Corridor In Hepatobiliary Surgery Department Of 2nd Floor". Movement time and sleep time are both displayed in a form of numerical value and time bar. A total length of the time bar for the movement time represents a whole day, and a filled part in the time bar represents a time period in which the human body moves. A numerical value of the movement time is a duration in which the human body moves. A total length of the time bar for the sleep time represents a duration from 8pm to 8am, and a filled part in the time bar represents a time period in which the human body sleeps. A numerical value of the sleep time is a total time length in which the human body sleeps. The EWS score is displayed in a form of a numerical value, specifically a numerical value "4" shown in a circular icon in the drawing.

In an embodiment of this disclosure, the first physiological parameter information may include multiple first main parameter data and at least one second main parameter data, wherein the first main parameter data is data of a numerical value type. In addition, the first physiological parameter information may include first auxiliary parameter data of a numerical value type and/or at least one data of a waveform type. Font(s) of multiple first main parameter data of the first physiological parameter information is(are) larger than a font of the at least one second main parameter data of the wear activity information. Optionally, the first main parameter data may be more important and/or more obvious than the second main parameter data in representing a physiological change of the human body. Therefore, the first main parameter data is displayed in a larger font for easy viewing.

In an embodiment of this disclosure, an interface displayed by the display 14 can be divided into different display areas, referring FIG. 5, can be divided into: a wear information area, a parameter area, a state bar and a hot key area, among which the wear information area is a first display area, and the parameter area is a second display area. The specific area division, a number of area, a size of each area, and information used to display, can be set according to actual requirements. The second display area includes multiple sub-display areas, at least some of the multiple sub-display areas have a same area, one sub-display area is configured to display first main parameter data, and an area, which is occupied by the first main parameter data displayed in each sub-display area, exceeds a preset threshold of an total area of said sub-display area. The specific preset threshold can be set according to actual requirements and application scenarios, and is not limited in the embodiments of this disclosure.

It should be noted that in the embodiments of this disclosure, the area occupied by the first main parameter data displayed in each sub-display area can exceed 40%, 50%, or even a larger proportion of the area of the corresponding sub-display area. In this way, the display of the first main parameter data in each sub-area is more prominent and easier to view.

In an embodiment of this disclosure, controlling the display 14, by the processor 12 in the bedside monitoring device 1, to distinguishingly display the first physiological parameter information and the wear activity information, includes: controlling the display 14 to distinguishingly display the first physiological parameter information and the wear activity information with different colors and/or different marks. Referring to FIG. 4, the first physiological parameter information and the wear activity information can be distinguishingly displayed in multiple colors and multiple marks. Specifically, the electrocardiogram (ECG) parameter can be a value "92" displayed in green, the blood pressure (NIBP) parameter can be a value "98" displayed in blue, the respiration (Resp) parameter can be a value "18" displayed in yellow, and the body temperature (Temp) parameter can be a value "36.8" displayed in white, so that medical staff can distinguish and check different information quickly. Optionally, the first physiological parameter information is displayed in a first color/first tone, and the wear activity information is displayed in a second color/second tone, the first color and the second color are different colors, and the first tone and the second tone are different tones. Optionally, the first physiological parameter information specifically includes different parameters which are distinguishingly displayed in different colors/different tones, and the wear activity information specifically includes different information which are also distinguishingly displayed in different colors/different tones.

In an embodiment of this disclosure, controlling the display 14, by the processor 12 in the bedside monitoring device 1, to distinguishingly display the first physiological parameter information and the wear activity information, includes: controlling the display 14 to display the wear activity information in the first display area and display the first physiological parameter information in the second display area.

It should be noted that in the embodiments of this disclosure, the first display area can be displayed at a top, bottom, left, and right of the interface, and can be folded and expanded; its position can be dragged at will, and the user can customize the first display area according to his or her preferences; display content of the first display area can be set in any combination; detailed information displayed by clicking on a target information item in the first display area can never block the first physiological parameter information displayed in the second display area.

It should be noted that, in the embodiments of this disclosure, the first display area and the second display area are in a left-right arrangement, or an up-down arrangement. Referring FIG. 5, the first display area and the second display area are displayed in a left-right arrangement, and the first display area is located on a left side of the second display area.

In an embodiment of this disclosure, the bedside monitoring device adjusts the arrangement of the first display area and the second display area based on an adjustment instruction inputted by a user; wherein the adjustment instruction includes followings.

When the first display area and the second display area are in the up-down arrangement, the adjustment instruction includes an instruction for adjusting an up-down position between the first display area and the second display area, or an instruction for adjusting the first display area and the second display area to be in the left-right arrangement.

When the first display area and the second display area are in the left-right arrangement, the adjustment instruction includes an instruction for adjusting a left-right position between the first display area and the second display area, or an instruction for adjusting the first display area and the second display area to be in the up-down arrangement.

It is understandable that, in the embodiments of this disclosure, the arrangement positions of the first display area and the second display area can be adjusted independently according to actual requirements. For example, the first display area and the second display area are in a left-right arrangement, wherein the first display area is on the left side and the second display area is on the right side, referring to FIG. 5. According to an instruction for adjusting the first display area from the left side to the right side, the first display area is adjusted from the left side to the right side, and correspondingly, the second display area is adjusted from the right side to the left side, on the interface displayed on the display 14.

It should be noted that, in the embodiment of this disclosure, the adjustment instruction may specifically be a movement operation, and the processor 12 in the bedside monitoring device 1 may adjust the arrangement position of the display area according to a movement trajectory of the movement operation. Exemplarily, referring to FIG. 5, the user can touch any position within the first display area displayed on the display 14, and then drag the first display area to the right side, thereby generating a movement trajectory from left to right, and the first display area will move from the left side to the right side following the movement trajectory, and accordingly, the second display area can adaptively move from the right side to the left side. In addition, the adjustment instruction may also be a click operation, a touch gesture, etc., and the specific adjustment instruction is not limited in the embodiment of this disclosure.

In an embodiment of this disclosure, the processor 12 in the bedside monitoring device 1 may control the display 14 to display detailed information of a target information item in the third display area, based on an operation instruction which is inputted by a user for the target information item, wherein the third display area partially or completely covers the first display area.

Alternatively, the processor 12 in the bedside monitoring device 1 may control the display 14 to display a part of the wear activity information in the first display area, and detailed information of a target information item in the first display area, based on an operation instruction which is inputted by a user for the target information item.

Alternatively, the processor 12 in the bedside monitoring device 1 may control the display 14 not display the wear activity information in the first display area, but to display detailed information of a target information item in the first display area, based on an operation instruction which is inputted by a user for the target information item.

Wherein, the target information item is any one item of the wear activity information.

It can be understood that in an embodiment of this disclosure, when the processor 12 in the bedside monitoring device 1 receives an operation instruction (for example, a selection operation) for any one item of the wear activity information, that is, for the target information item, the processor 12 can control the display 14 to display the detailed information of the target information item in a third display area which covers part or all of the first display area. Optionally, the third display area has a same area as that of the first display area and completely covers the first display area, and the wear activity information is blocked. After the medical staff views the detailed information of the target information item, the processor 12 can control the display 14 to stop displaying the third display area. At this time, the first display area will not be covered, so that the medical staff can continue to view the wear activity information displayed in the first display area. Optionally, the processor 12 may control the display 14 to stop displaying the third display area when a display time length of the third display area reaches a certain time length, or may control the display 14 to stop displaying the third display area according to an instruction to stop displaying the third display area, when receiving said instruction, and the embodiment of this disclosure is not limited to this.

It can be understood that in the embodiment of this disclosure, when the processor 12 in the bedside monitoring device 1 receives an operation instruction for a target information item, it can also control the display 14 to display a part of the wear activity information in the first display area, and to display the detailed information of the target information item in the first display area. In this way, while viewing the detailed information of the target information item, medical staff can also view a part of the wear activity information, and the first physiological parameter information, at the same time, which is convenient for medical staff to determine the physical condition of the human body more accurately. Wherein, the part of the wear activity information displayed in the first display area can be set independently, or can be selected according to an importance of information. For example, a part of the wear activity information with higher importance is selected for displaying, which is not limited in the embodiment of this disclosure.

It can be understood that in the embodiment of this disclosure, when the processor 12 in the bedside monitoring device 1 receives an operation instruction for the target information item, it can also control the display 14 not to display the wear activity information in the first display area, and display the detailed information of the target information item in the first display area. At this time, the first physiological parameter information displayed in the second display area will not be blocked, and medical staff can view the first physiological parameter information at the same time, when viewing the detailed information of the target information item.

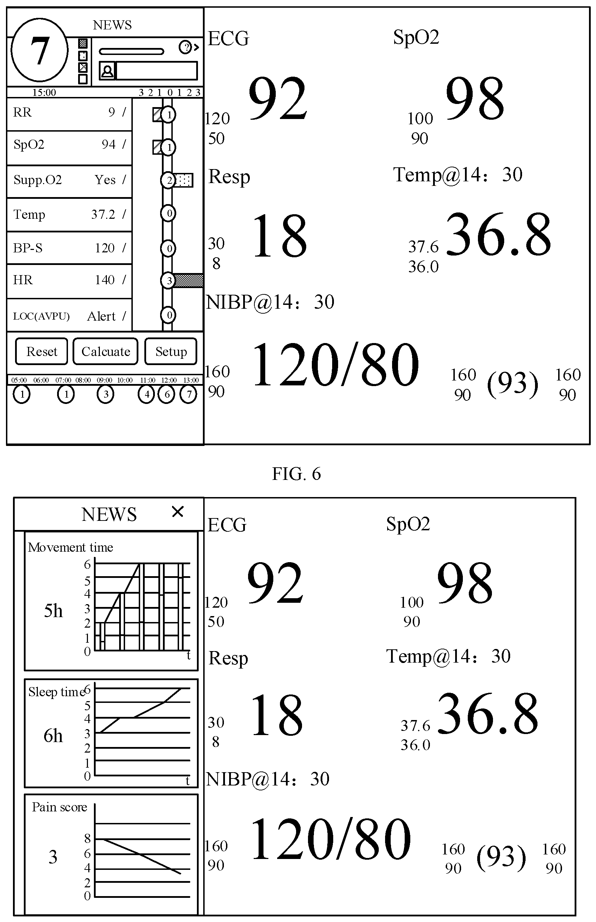

Exemplarily, in an embodiment of this disclosure, referring FIG. 4, the wear activity information displayed in the first display area includes an EWS score. When the processor 12 in the bedside monitoring device 1 receives an operation instruction for the EWS score, it controls the display 14 not to display, in the first display area, original information displayed in the first display area, and but to display detailed information of the EWS score in the first display area, referring FIG. 6, detailed information of the EWS score specifically includes a respiratory (RR) parameter, a blood oxygen saturation (SpO2) parameter, an oxygen concentration (Supp.O2) parameter, a body temperature (Temp) parameter, a systolic blood pressure (BP-S) parameter, a consciousness state LOC (AVPU) parameter, wherein each parameter corresponds to a sub-score, wherein the consciousness state LOC (AVPU) parameter is specifically Alert, indicating that the human body is sensitive to reaction, and the sub-score of each parameter is displayed in a form of a bar, and a length of the bar is associated with a specific value of the sub-score, the longer the bar, the larger the value, and a direction of the bar can reflect a trend of change for the sub-score relative to a benchmark threshold, and a standard scale for the sub-score can also be displayed above the sub-parameter and the sub-score. Optionally, the displayed information may be closed based on a user operation, thereby continuing to display the original information in the first display area. In addition, referring to FIG. 6, a manual calculation (Calculate) icon can also be displayed in the first display area, by clicking which, a calculation on the EWS can be started at any time; a setup (Setup) icon can also be displayed, by clicking which, a property setting window can be entered to select other functions, implement page settings, and mode settings; a reset (Reset) icon can also be displayed; a trend chart of historical early warning state score for the human body can also be displayed, that is, a timeline is displayed in the first display area and different scores are marked at different time points on the time line; a real-time state icon of the human body can also be displayed at the top, i.e., an icon formed by a circle and a value "7" above the first display area.

Exemplarily, in an embodiment of this disclosure, referring to FIG. 4, the wear activity information displayed in the first display area includes sleep time. When the processor 12 in the bedside monitoring device 1 receives an operation instruction for the sleep time, it controls the display 14 not to display, in the first display area, original information displayed in the first display area, but display, in the first display area, detailed information of the sleep time. Referring to FIG. 7, a curve graph of sleep time can be displayed. In addition, a control element for the sleep time is associated with the movement time and a detailed page for the sleep time. Accordingly, FIG. 7 can also display a bar graph of the movement time. Moreover, since time lengths, in which the human body moves and sleeps, can reflect a degree of pain in the human body, the detailed information displayed also includes a pain score of the human body.

In an embodiment of this disclosure, referring to FIG. 4, when at least posture information and other information of the human body are displayed in the first display area, the posture information and other information are in an up and down arrangement; said other information includes at least one of position information, movement time, sleep time and early-warning state evaluation, wherein the early-warning state evaluation is determined by the bedside monitoring device, through receiving the physiological parameter signal acquired by the mobile monitoring device and at least processing the physiological parameter signal, or the early-warning state evaluation is generated by the mobile monitoring device and then received by the bedside monitoring device.

It should be noted that, in the embodiment of this disclosure, when the first display area at least displays the posture information and other information of the human body, the posture information and said other information may also be in a left-right arrangement.

It should be noted that, in the embodiment of this disclosure, an area occupied by the posture information is not less than a preset proportion of a total area of the first display area, such as 1/3, 2/5, etc.

In an embodiment of this disclosure, in the above-mentioned monitoring system, a display scheme of the bedside monitoring device 1 is that the display mode of the bedside monitoring device 1 can correspond to the monitoring mode of the mobile monitoring device 2. Different display modes correspond to different monitoring modes, which are described in detail below.

In an embodiment of this disclosure, referring to FIG. 1, the monitoring system includes: a bedside monitoring device 1, and a mobile monitoring device 2 which is capable of being worn by a human body.

A display mode of the bedside monitoring device 1 includes a first display mode and a second display mode.

A monitoring mode of the mobile monitoring device 2 includes a first monitoring mode and a second monitoring mode.

A frequency of the mobile monitoring device 2 when operating in the first monitoring mode is lower than a frequency of the mobile monitoring device 2 when operating in the second monitoring mode; a frequency of the mobile monitoring device 2 when operating in the first monitoring mode being lower than a frequency of the mobile monitoring device 2 when operating in the second monitoring mode, includes at least one of following two situations: a signal acquisition frequency of the mobile monitoring device 2 when operating in the first monitoring mode is lower than a signal acquisition frequency of the mobile monitoring device 2 when operating in the second monitoring mode, and a data transmission frequency of the mobile monitoring device 2 when operating in the first monitoring mode is lower than a data transmission frequency of the mobile monitoring device 2 when operating in the second monitoring mode.

When the display mode of the bedside monitoring device 1 is the first display mode and the mobile monitoring device 2 operates in the first monitoring mode, the bedside monitoring device 1 is configured to obtain first physiological parameter information and wear activity information, and distinguishingly display the first physiological parameter information and the wear activity information.

When the display mode of the bedside monitoring device 1 is the second display mode and the mobile monitoring device 2 operates in the second monitoring mode, the bedside monitoring device 1 is configured to at least obtain second physiological parameter information, and display the second physiological parameter information.

The first physiological parameter information and the second physiological parameter information are obtained by the bedside monitoring device 1 through receiving and processing a physiological parameter signal of the human body acquired by the mobile monitoring device 2, or by the bedside monitoring device 1 through receiving physiological parameter information of the human body generated by the mobile monitoring device 2.

The wear activity information is obtained by the bedside monitoring device 1 through receiving and at least processing a non-physiological parameter signal acquired by the mobile monitoring device 2, or by the bedside monitoring device 1 through receiving the wear activity information of the human body generated by the mobile monitoring device 2.

Wherein the wear activity information includes at least one of: posture information, position information, movement time, and sleep time, of the human body.

It should be noted that, in the embodiment of this disclosure, in the monitoring mode of the mobile monitoring device 2, the first monitoring mode is actually a wearable mode in which the mobile monitoring device 2 performs a human body monitoring when the human body moves within a large range, and the second monitoring mode is actually a continuous mode in which the human body is basically in a specific area, for example, in a ward, and the mobile monitoring device 2 performs a human body monitoring when the human body moves within a small range.

It should be noted that, in the embodiment of this disclosure, the first display mode of the bedside monitoring device 1 corresponds to that the mobile monitoring device 2 operates in the first monitoring mode; the second display mode of the bedside monitoring device 1 corresponds to that the mobile monitoring device 2 operates in the second monitoring mode. The mobile monitoring device 2 may determine the monitoring mode and notify the bedside monitoring device 1; when the mobile monitoring device 2 operates in the first monitoring mode, the bedside monitoring device 1 displays in the first display mode; when the mobile monitoring device 2 operates in the second monitoring mode, the bedside monitoring device 1 displays in the second display mode. Alternatively, when the bedside monitoring device 1 determines that the mobile monitoring device 2 needs to operate in the first monitoring mode/the second monitoring mode, the bedside monitoring device 1 notifies/controls the mobile monitoring device 2 to operate in the corresponding monitoring mode. Optionally, after notification or after control, the bedside monitoring device 1 may adjust its own display mode to correspond to the monitoring mode of the mobile monitoring device 2. For example, the user controls a required display mode on the bedside monitoring device 1, and then notifies the mobile monitoring device 2 of the monitoring mode in which the mobile monitoring device 2 operates; when the bedside monitoring device 1 is controlled to display in the first display mode, the mobile monitoring device 2 is notified to operate in the first monitoring mode, and when the bedside monitoring device 1 is controlled to display in the second display mode, the mobile monitoring device 2 is notified to operate in the second monitoring mode.

It can be understood that in the embodiments of this disclosure, referring to FIG. 2 and 3, the bedside monitoring device 1 includes a communication unit 11, and the mobile monitoring device 2 includes a communication unit 24. When the mobile monitoring device 2 operates in the first monitoring mode and the second monitoring mode, information is transmitted to the communication unit 11 of the bedside monitoring device 1 through the communication unit 24.

In an embodiment of this disclosure, the first physiological parameter information includes at least physiological parameter information of a numerical value type, and the second physiological parameter information includes physiological parameter information of a numerical value type and a waveform type.

Exemplarily, in an embodiment of this disclosure, as shown in FIG. 4, when the display mode of the bedside monitoring device 1 is the first display mode, the first physiological parameter information displayed includes: an electrocardiogram (ECG) parameter, a respiration (Resp) parameter, a blood oxygen saturation (SpO2) parameter, a blood pressure (NIBP) parameter, and a body temperature (Temp) parameter. These physiological parameter are only displayed in a numerical form. Optionally, when the display mode of the bedside monitoring device 1 is the first display mode, it also includes an early-warning state evaluation of the human body, such as an EWS score.

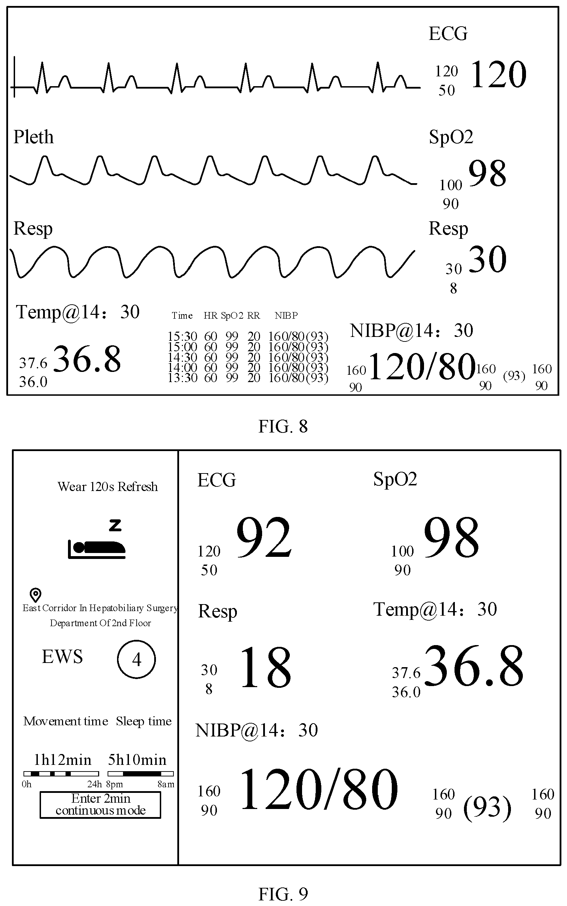

Exemplarily, in an embodiment of this disclosure, as shown in FIG. 8, when the display mode of the bedside monitoring device 1 is the second display mode, the second physiological parameter information displayed may include: an electrocardiogram (ECG) parameter, a respiration (Resp) parameter, a blood oxygen saturation (SpO2) parameter, a blood pressure (NIBP) parameter, as well as a body temperature (Temp) parameter and a pulse volume (Pleth) parameter, wherein these parameter are displayed in a numerical form and/or a waveform form. For example, in FIG. 8, the ECG parameter is specifically displayed in a form of an ECG waveform and of an ECG value, and the temperature (Temp) parameter is specifically displayed in a form of a numerical value, that is, the displayed numerical value "36.8". In addition, as shown in FIG. 8, some other relevant information of these parameters is also displayed, which can be set according to actual requirements and is not limited in the embodiment of this disclosure. For example, above the body temperature (Temp) parameter "36.8", time information "14:30" of the body temperature (Temp) parameter is displayed. As shown in FIG. 8, the display 14 can also display the historical trend information of the second physiological parameter information in a numerical form, specifically displaying a heart rate (HR) parameter, a blood oxygen saturation (SpO2) parameter, a respiratory rate (RR) parameter and a blood pressure (NIBP) parameter, obtained every 30 minutes from 13:30 to 15:30.

In an embodiment of this disclosure, the wear activity information may include posture information, and the posture information includes at least one of: sleep state information, lying state information, movement state information, fall state information, and stand state information; the bedside monitoring device 1 displays different posture information in different icons and/or different text. For specific display forms of different posture information, please refer to FIGS. 4 and 9 to 11.

In the embodiment of this disclosure, the posture information includes normal posture information and abnormal posture information; an indication intensity which corresponds to the abnormal posture information displayed by the bedside monitoring device 1 is higher than an indication intensity which corresponds to the normal posture information displayed by the bedside monitoring device 1.

It should be noted that, for different human bodies, their normal posture information and abnormal posture information may be different. For example, for a certain human body, its abnormal posture information may be fall state information, and its normal posture information may be lying state information, sleep state information, movement state information, etc. The embodiment of this disclosure is not limited to this. The abnormal posture information can be displayed with a higher indication intensity than the normal posture information, including but not limited to a larger font, a more obvious main color of icon, or animation, etc.

In an embodiment of this disclosure, when the display mode of the bedside monitoring device 1 is the first display mode and the mobile monitoring device 2 operates in the first monitoring mode, if the posture information of the human body is fall state information, the bedside monitoring device 1 displays first indication information which corresponds to the fall state information, wherein the first indication information includes icon(s) and/or text which correspond/corresponds to the fall state information, and includes real-time position information of the human body.

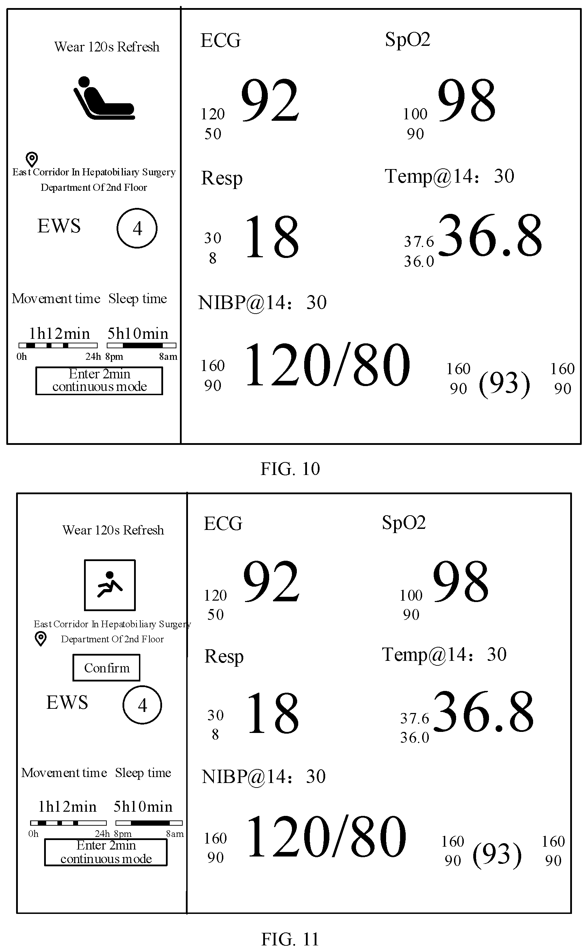

It should be noted that in an embodiment of this disclosure, referring to FIG. 11, an icon to represent a fall of the human body can be displayed as a flashing, animated icon or an icon with a special color, and real-time position information of the human body can also be displayed, so as to enable the medical staff to check more promptly and provide timely assistance when the patient falls. In addition, referring to FIG. 11, indication information corresponding to the fall state information may also include a "confirm" icon, and the medical staff may confirm that the human body is in a fall state by clicking on the icon. If the icon is not clicked, the icon can be continuously displayed, or can be cancelled displaying after a certain time period, and a reminder can be given in other forms, such as playing an alarm tone, etc., which is not limited in the embodiments of this disclosure.

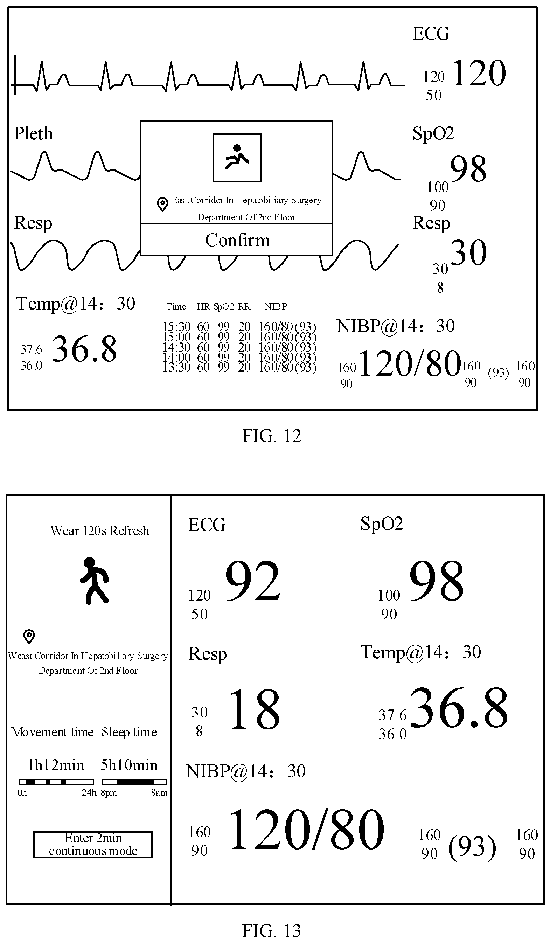

It should be noted that in an embodiment of this disclosure, referring to FIG. 12, when the display mode of the bedside monitoring device 1 is the second display mode and the mobile monitoring device 2 operates the second monitoring mode, while displaying the second physiological parameter information, if the posture information of the human body is fall state information, the bedside monitoring device 1 may also display first indication information corresponding to the fall state.

In an embodiment of this disclosure, when the display mode of the bedside monitoring device 1 is the first display mode and the mobile monitoring device 2 operates in the first monitoring mode, the bedside monitoring device 1 obtains an early-warning state evaluation of the human body and displays the early-warning state evaluation; wherein the early-warning state evaluation is determined by the bedside monitoring device 1 through receiving a physiological parameter signal acquired by the mobile monitoring device 2 and at least processing the physiological parameter signal, or by the bedside monitoring device 1 through receiving the early-warning state evaluation of the human body which state evaluation is generated by the mobile monitoring device 2.

In an embodiment of this disclosure, the early-warning state evaluation includes but is not limited to EWS statistical information, wherein the EWS statistical information includes at least one of: a current EWS score, sub-scores and measurement values of N parameters corresponding to the EWS score, and a trend of change for EWS score, where N is a natural number greater than or equal to 1. Referring FIG. 4, in which a numerical value "4" displayed in the circular icon on the left side is the EWS score.

It should be noted that, in the embodiments of this disclosure, the EWS score mainly assigns corresponding scores to some physiological parameter information, and then uses statistical values of the scores to evaluate a score that represents a clinical state or potential risk of the patient. Medical staff can take different treatment measures for patients based on different EWS scores. For example, a score of 0 to 5 indicates that a medical condition of the patient is stable and there is no risk of potential critical illness. The patient can be treated according to general routine procedures. A score of 5 to 8 indicates that the medical condition of the patient is unstable and changes greatly. The patient has a potential critical illness and should be given priority treatment. The patient should be informed of the relevant situation in a timely manner and should be arranged to be admitted to a specialist ward or even an intensive care unit in a timely manner. A score greater than 9 indicates that the medical condition of the patient is critical and the risk of death is significantly increased. If conditions permit, the patient should be immediately sent to an intensive care unit or a specialist ward for treatment.

In an embodiment of this disclosure, the measured values of the above-mentioned N parameters may be values obtained through physiological parameter information of the human body. Scoring rules corresponding to various parameters may be stored in the monitoring device. For example, if a heart rate parameter is between 51 and 100, a corresponding score may be 0, and if a heart rate is between 41 and 50 or 101 and 110, a corresponding score is 1. The EWS score is determined by scores of N parameters. For example, the EWS score is determined by five scores corresponding to five parameters. The specific number and type of parameter are not limited in this embodiment of the application.

It should be noted that, in the embodiment of this disclosure, the early-warning state evaluation may be displayed in the first display area. Of course, it is also possible to determine not to display the early-warning state evaluation based on actual requirements and an interface size of the display 14. Referring to FIG. 13, the EWS score is not displayed.

In an embodiment of this disclosure, distinguishingly displaying, by the bedside monitoring device 1 described above, the first physiological parameter information and the wear activity information, is specifically executed, when the display mode of the bedside monitoring device 1 is the first display mode and the mobile monitoring device 2 operates in the first monitoring mode. The specific distinguishing display method may be displaying the wear activity information in the first display area and displaying the first physiological parameter information in the second display area as described above, or may be distinguishingly displaying the wear activity information and the first physiological parameter information with different colors and/or different marks, as described above, which will not be repeated here.

In an embodiment of this disclosure, an area which displays the second physiological parameter information in the second display mode is a fourth display area.

Respective areas occupied by the second display area in the first display mode and by the fourth display area in the second display mode are at least partially same on the display 14; and/or,
in the first display mode, an area occupied by the first display area is smaller than an area occupied by the second display area; and/or,
a total area occupied by the first display area and the second display area on the display 14 in the first display mode is same as an area occupied by the fourth display area on the display 14 in the second display mode.

Exemplarily, in an embodiment of this disclosure, referring to FIG. 4, in the first display mode, an area of the first display area is smaller than an area of the second display area. For another example, referring to FIG. 4 and FIG. 8, the fourth display area for displaying the second physiological parameter information in the second display mode is the same as a total area occupied by the first display area and the second display area in the first display mode.

In an embodiment of this disclosure, the bedside monitoring device 1 may determine its display mode based on specific conditions, which will be described in detail below.

In an embodiment of this disclosure, when a first preset condition is satisfied, the bedside monitoring device 1 determines that its display mode is the first display mode; the first preset condition includes at least one of followings.

A first instruction for instructing the bedside monitoring device to display in a first display mode is received, which instruction is inputted by a user on the bedside monitoring device 1. A second instruction for instructing the bedside monitoring device to display in the first display mode is received, which instruction is transmitted by the mobile monitoring device 2. An indicator, which reflects a communication state between the mobile monitoring device 2 and the bedside monitoring device 1, satisfies a first indicator condition. An indicator, which reflects a health state of the human body, satisfies a second indicator condition.

Wherein if the mobile monitoring device 2 notifies the bedside monitoring device 1 to operate in the first monitoring mode, this notification can serve as a second instruction for instructing the bedside monitoring device 1 to display in the first display mode; or, if the mobile monitoring device 2 transmits data at a higher frequency, so as to transmit a physiological parameter signal and/or a non-physiological parameter signal to the bedside monitoring device 1, such transmission can serve as a second instruction for instructing the bedside monitoring device 1 to display in the first display mode.

In an embodiment of this disclosure, an indicator, which reflects a communication state between the mobile monitoring device 2 and the bedside monitoring device 1, satisfying a first indicator condition, includes at least one of: a communicative connection between the mobile monitoring device 2 and the bedside monitoring device 1 is not a point-to-point wireless communicative connection; when the bedside monitoring device 1 is located inside a ward, the mobile monitoring device 2 is located outside said ward; a communication distance between the mobile monitoring device 2 and the bedside monitoring device 1 is greater than a first preset distance threshold.

It should be noted that, in the embodiments of this disclosure, the connection, which is not a point-to-point wireless communicative connection, may include a wireless communicative connection via an access point (AP) network connection. Since the connection, which is not a point-to-point wireless communicative connection, consumes a lot of power, in this case, the first monitoring mode with a lower transmission frequency/acquisition frequency can be used to save power consumption, and the corresponding bedside monitoring device 1 can display in the first display mode.

It can be understood that in the embodiment of this disclosure, the bedside monitoring device 1 is a monitor located inside the ward, and the mobile monitoring device 2 is located outside said ward. The communication distance between the bedside monitoring device 1 and the mobile monitoring device 2 is far, and a higher transmission frequency/acquisition frequency will result in too much power consumption. Therefore, in this case, the first monitoring mode with a lower transmission frequency/acquisition frequency can be used to save power consumption, and the corresponding bedside monitoring device 1 can display in the first display mode. In addition, the mobile monitoring device 2 is located outside said ward, that is, the patient wearing it has walked out of the ward, which may mean that the medical condition of the patient has improved. At this time, there is no need to use the second monitoring mode with a higher transmission frequency/acquisition frequency, and the first monitoring mode with a lower transmission frequency/acquisition frequency can be used. The corresponding bedside monitoring device 1 can display in the first display mode.

The distance between the mobile monitoring device 2 and the bedside monitoring device 1 is greater than the first preset distance threshold, which is similar to a situation that the bedside monitoring device 1 is inside the ward and the mobile monitoring device 2 is outside the ward. Due to the large distance between the mobile monitoring device 2 and the bedside monitoring device 1, the communication distance is long, and a higher transmission frequency/acquisition frequency will result in too much power consumption. Therefore, in this case, the first monitoring mode with a lower transmission frequency/acquisition frequency can be used to save power consumption, and the corresponding bedside monitoring device 1 can display in the first display mode. In addition, the mobile monitoring device 2 is far away from the bedside monitoring device 1. If the bedside monitoring device 1 is a monitor inside the ward, it also means that the mobile monitoring device 2 may be located outside said ward, that is, the patient wearing it has walked out of the ward, which may mean that the medical condition of the patient has improved. At this time, there is no need to use the second monitoring mode with a higher transmission frequency/acquisition frequency. The first monitoring mode with a lower transmission frequency/acquisition frequency can be used, and the corresponding bedside monitoring device 1 can display in the first display mode.

In an embodiment of this disclosure, an indicator, which reflects a health state of a human body satisfying a second indicator condition, includes at least one of: a time length for the mobile monitoring device 2 to monitor the human body in the second monitoring mode reaches a first time threshold; an early-warning state evaluation of the human body is higher than a first evaluation threshold, wherein the early-warning state evaluation is determined at least based on a received physiological parameter signal, or the early-warning state evaluation is transmitted by the mobile monitoring device 2 and obtained by the mobile monitoring device 2 at least based on a physiological parameter signal; at least one item of physiological parameter information of the human body has no abnormity at a current moment and/or within a preset historical time period; posture information of the human body is normal state information.

In this embodiment, at least one of the monitoring time, the EWS score based on the monitoring data, the monitoring data itself, and other unexpected event is used as an indicator which reflects a health state of the human body. Wherein, a monitoring time, for example, within 8 hours after surgery is a relatively important monitoring time. During this time, the patient needs to be closely monitored to ensure that the patient is monitored for possible postoperative risks. After this time, the medical condition is generally stable. Therefore, after the mobile monitoring device 2 has monitored the patient for 8 hours, the first monitoring mode with a lower transmission frequency/acquisition frequency may be used. Of course, this is merely exemplary, and in other cases, other time thresholds (collectively referred to as a first time threshold) may be used for determination.

In addition, the EWS score based on the monitoring data can reflect an overall medical condition of the patient. When the EWS score is higher than the first evaluation threshold, the overall medical condition of the patient can be considered stable or good. Therefore, the first monitoring mode with a lower transmission frequency/acquisition frequency should be used at this time.

In addition, the absence of abnormalities in the monitoring data generally indicates that overall medical condition of the patient is stable or good. Therefore, the first monitoring mode with a lower transmission frequency/acquisition frequency should be used at this time. Similarly, if the patient does not have any movement accident, there is no need to adopt the second monitoring mode with a higher transmission frequency/acquisition frequency, and the first monitoring mode with a lower transmission frequency/acquisition frequency can be used.

In an embodiment of this disclosure, when a second preset condition is satisfied, the bedside monitoring device 1 determines that its display mode is the second display mode; the second preset condition includes at least one of followings. A third instruction for instructing the bedside monitoring device to display in a second display mode is received, which instruction is inputted by a user on the bedside monitoring device 1. A fourth instruction for instructing the bedside monitoring device to display in the second display mode, which instruction is transmitted by the mobile monitoring device 2. An indicator, which reflects a communication state between the mobile monitoring device 2 and the bedside monitoring device 1, satisfies a third indicator condition. An indicator, which reflects a health state of the human body, satisfies a fourth indicator condition.

Wherein, if the mobile monitoring device 2 notifies the bedside monitoring device 1 to operate in the second monitoring mode, this notification can serve as a third instruction which instructs the bedside monitoring device 1 to display in the second display mode; or, if the mobile monitoring device 2 transmits data at a lower frequency, so as to transmit a physiological parameter signal and/or a non-physiological parameter signal to the bedside monitoring device 1, such transmission can serve as a third instruction which instructs the bedside monitoring device 1 to display in the second display mode.

In an embodiment of this disclosure, the bedside monitoring device 1 may display a control element for a switch operation; when an operation on the control element for a switch operation is detected, a third instruction which instructs to display in the second display mode is received, which instruction is inputted by a user on the bedside monitoring device 1.

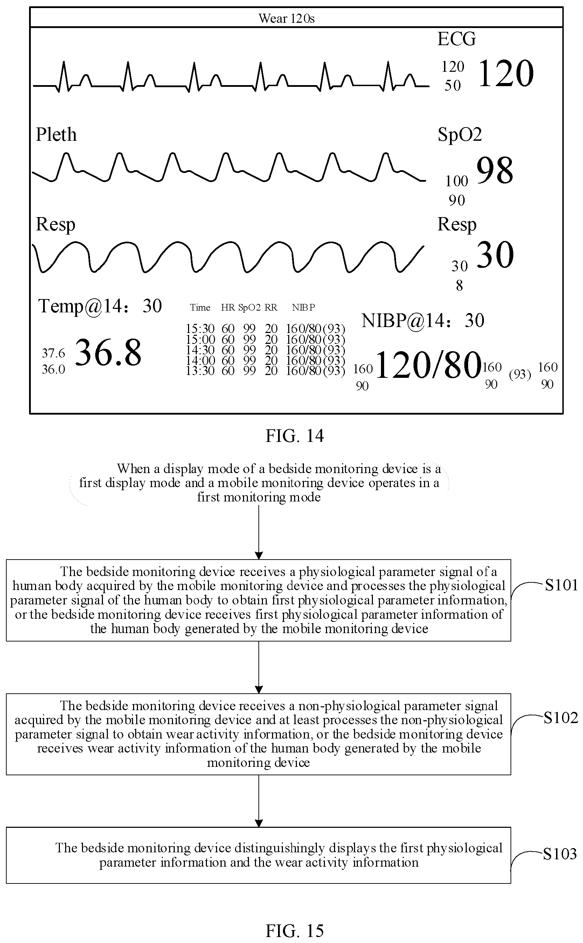

It should be noted that in the embodiment of this disclosure, referring to FIG. 4, the control element for a switch operation is specifically a control element of "enter 2min continuous mode" in the drawing. When the bedside monitoring device 1 detects an operation on the control element, the third instruction is received, and a display content in the first display mode shown in FIG. 4 is switched to a display content in the second display mode shown in FIG. 14.

It should be noted that, in an embodiment of this disclosure, after the display mode of the bedside monitoring device 1 is switched from the first display mode to the second display mode, if a time length passed reaches the first preset time threshold and the second preset condition is not satisfied, the display mode of the bedside monitoring device 1 is switched back to the first display mode. For example, after the display mode of the bedside monitoring device 1 is switched from the first display mode to the second display mode, if 120 seconds have passed and the second preset condition is not satisfied, the second display mode is switched back to the first display mode. In addition, a countdown indication for switching back to the first display mode may be displayed in the second display mode, namely, "Wear 120s" as shown in FIG. 14.

In an embodiment of this disclosure, the indicator, which reflects a communication state between the mobile monitoring device 2 and the bedside monitoring device 1, satisfying a third indicator condition, includes at least one of: a communicative connection between the mobile monitoring device 2 and the bedside monitoring device 1 is a point-to-point wireless communicative connection; when the bedside monitoring device 1 is located inside a ward, the mobile monitoring device 2 is also located inside said ward; a communication distance between the mobile monitoring device 2 and the bedside monitoring device 1 is not greater than a second preset distance threshold.

The point-to-point wireless communicative connection involved in the embodiments of this disclosure may include a Bluetooth connection, a Wireless Fidelity (WIFI) direct connection, a Wireless Medical Telemetry Services (WMTS) direct connection, etc. Since the point-to-point communicative connection has lower power consumption, the mobile monitoring device will not consume too much power even if the transmission frequency/acquisition frequency is high, so the mobile monitoring device 2 can operate in the second monitoring mode. Therefore, in this case, the bedside monitoring device 1 can use the second display mode accordingly.

It can be understood that in the embodiment of this disclosure, the bedside monitoring device 1 is a monitor located inside a ward, and the mobile monitoring device 2 is also located inside said ward. Since both the mobile monitoring device 2 and the bedside monitoring device 1 are inside the same ward, the communication distance between the mobile monitoring device 2 and the bedside monitoring device 1 is short, and even if the transmission frequency/acquisition frequency is high, there will not be too much power consumption. Therefore, in this case, the mobile monitoring device 2 can operate in the second monitoring mode, and the bedside monitoring device 1 can use the second display mode accordingly. In addition, the mobile monitoring device 2 is located inside the ward, that is, the patient wearing it has not left the ward, which may mean that the medical condition of the patient is not suitable for getting out of bed. At this time, it is necessary to use the second monitoring mode with a higher transmission frequency/acquisition frequency to ensure a monitoring safety.

The distance between the mobile monitoring device 2 and the bedside monitoring device 1 is not greater than the second preset distance threshold, which is similar to a situation that both of the mobile monitoring device 2 and the bedside monitoring device 1 are inside the same ward. Since the distance between the mobile monitoring device 2 and the bedside monitoring device 1 is small, the communication distance is short, even if the transmission frequency/acquisition frequency is high, there will not be much power consumption. Therefore, in this case, the mobile monitoring device 2 can operate in the second monitoring mode, and the bedside monitoring device 1 can use the second display mode accordingly. In addition, the distance between the mobile monitoring device 2 and the bedside monitoring device 1 is small. If the bedside monitoring device 1 is a monitor inside the ward, it also means that the mobile monitoring device 2 is also located inside said ward, that is, the patient wearing it has not walked out of the ward. This may mean that the medical condition of the patient is not suitable for getting out of bed. At this time, it is necessary to use the second monitoring mode with a higher transmission frequency/acquisition frequency to ensure a monitoring safety.

In an embodiment of this disclosure, an indicator, which reflects a health state of a human body, satisfying a fourth indicator condition, includes at least one of: a time length for the mobile monitoring device to monitor the human body in a first monitoring mode reaches a second time threshold; an early-warning state evaluation of the human body is lower than a second evaluation threshold, wherein the early-warning state evaluation is determined at least based on a received physiological parameter signal, or the early-warning state evaluation is transmitted by the mobile monitoring device and obtained by the mobile monitoring device at least based on a physiological parameter signal; at least one item of physiological parameter information of the human body has an abnormity at a current moment and/or within a preset historical time period; posture information of the human body is abnormal state information.

In this embodiment, at least one of the monitoring time, the EWS score based on the monitoring data, the monitoring data itself, and other unexpected event is used as an indicator which reflects a health state of the human body. Wherein, a monitoring time, for example, within 8 hours after surgery is a relatively important monitoring time. During this time, the patient needs to be closely monitored to ensure that the patient is monitored for possible postoperative risks. Therefore, when the monitoring time of the patient by mobile monitoring device is less than 8 hours, the second monitoring mode with higher transmission frequency/acquisition frequency should be used. Of course, this is merely exemplary, and in other cases, other time thresholds (collectively referred to as a second time thresholds) may be used for determination.

In addition, the EWS score based on monitoring data can reflect an overall medical condition of the patient. When the EWS score is lower than the second evaluation threshold, the overall medical condition of the patient can be considered as still requiring more monitoring data to ensure safety. Therefore, the second monitoring mode with a higher transmission frequency/acquisition frequency should be used at this time.

In addition, some important physiological parameter information (such as heart rate, blood oxygen saturation, etc.), which may reflect a change in the medical condition when having an abnormality, should be closely monitored immediately. For such cases, a second monitoring mode with a higher transmission frequency/acquisition frequency should also be used.

In addition, for such physiological parameter information, even if its abnormality disappears after a period of monitoring or treatment and begins to no longer be abnormal, close monitoring can be continued for a time period to ensure that the situation without such abnormality is maintained for a time period before temporarily considering that the medical condition is stable and no longer requires close monitoring. This can further improve the monitoring safety. In this case, that is, at least one item of physiological parameter information has no abnormality at a current moment, but has an abnormality within a preset time before the current moment, it is still necessary to use the second monitoring mode with a higher transmission frequency/acquisition frequency. That is, the transmission mode is not switched immediately after the abnormality disappears, but data is transmitted in the second monitoring mode with a higher transmission frequency/acquisition frequency within a preset time, and then the second monitoring mode is switched back to the first monitoring mode after the preset time is over.

In addition, when an unexpected patient movement accident occurs, such as a patient falling, the patient may fall because of an abnormality, or an abnormality may occur due to the fall. Therefore, a second monitoring mode with a higher transmission frequency/acquisition frequency should be used at this time to ensure a monitoring safety. After the monitoring data is transmitted in the second monitoring mode for a time period, if no abnormality is detected or if an abnormality existed but has disappeared, the second monitoring mode can be switched back to the first monitoring mode.

Exemplarily, in an embodiment of this disclosure, the first indication information can specifically be a countdown indication, and does not block displaying any other information. The countdown indication at least includes a countdown value. Of course, the countdown indication can also include a specific mark, and a start value of the countdown value can be a numerical value of a certain time length.

It is understandable that in the embodiment of this disclosure, the second preset condition needs to be continuously satisfied within a preset time period, so as to switch from the first display mode to the second display mode after the preset time period. If the second preset condition is not satisfied within the preset time period, a timing will be stopped at a moment when the second preset condition is not satisfied, and the display mode will not be switched from the first display mode to the second display mode, so as to ensure a switching accuracy of the display mode. For example, the bedside monitoring device 1 being located inside the ward, and the communicative connection between the mobile monitoring device 2 and the bedside monitoring device 1 not being a point-to-point wireless communicative connection, actually represent that the human body may move from an inside of the ward to an outside of the ward. However, in actual application scenarios, if the human body only returns into the ward from the outside of the ward for a short time and then moves outside the ward, it is actually not necessary to switch from the first display mode to the second display mode during this period. The setting of the preset time period and the detection for whether the second preset condition is continuously satisfied within the preset time period can avoid frequent switching of the display mode.

In an embodiment of this disclosure, the bedside monitoring device 1 may further display a countdown indication from a refreshed display for next time; wherein the countdown indication includes at least a numerical countdown value.

Exemplarily, in an embodiment of this disclosure, referring to FIGS. 4, 9 to 11, and 13, a countdown indication for a refresh display is "Wear 120s Refresh" in the first display area, which does not block any information, that is, is displayed in a blank display position.

It should be noted that, in an embodiment of this disclosure, the bedside monitoring device 1 can also transmit the acquired wear activity information, the first physiological parameter information, or the second physiological parameter information to the mobile terminal for displaying on a mobile terminal.

The bedside monitoring device 1 provided in the embodiment of this disclosure can display monitoring information in different display modes according to different monitoring modes of the mobile monitoring device 2, support diverse monitoring display, and improve a monitoring display effect.

An embodiment of this disclosure provides a bedside monitoring device 1. Referring to the structure of the bedside monitoring device 1 shown in FIG. 2, the bedside monitoring device 1 includes: a communication unit 11, a processor 12, a memory 13, and a display 14.

The communication unit 11 is in wireless communication with a mobile monitoring device 2 which is capable of being worn by a human body.

The communication unit 11 is configured to receive a physiological parameter signal of the human body acquired by the mobile monitoring device 2 and the processor 12 is configured to process the physiological parameter signal of the human body to obtain physiological parameter information; or the communication unit 11 is configured to receive physiological parameter information of the human body generated by the mobile monitoring device 2.

The communication unit 11 is further configured to receive a non-physiological parameter signal acquired by the mobile monitoring device 2, and the processor 12 is configured to process the non-physiological parameter signal to obtain wear activity information, or the communication unit 11 is further configured to receive wear activity information of the human body generated by the mobile monitoring device 2.

The memory 13 is configured to store executable instructions, and the processor 12 is configured to execute the executable instructions, so as to perform following operations:
when a display mode of the bedside monitoring device 1 is a first display mode, obtaining first physiological parameter information and the wear activity information, and controlling the display 14 to distinguishingly display the first physiological parameter information and the wear activity information.
when a display mode of the bedside monitoring device 1 is a second display mode, obtaining at least second physiological parameter information, and controlling the display 14 to display the second physiological parameter information.

Wherein the wear activity information includes at least one of: posture information, position information, movement time, and sleep time, of the human body.

In an embodiment of this disclosure, the processor 12 is configured to control the display 14 to display the first physiological parameter information and the wear activity information in different colors and/or different marks.

In an embodiment of this disclosure, controlling the display 14, by the processor 12, to distinguishingly display the first physiological parameter information and the wear activity information, includes: controlling the display 14 to display the wear activity information in a first display area, and displaying the first physiological parameter information in a second display area.

In an embodiment of this disclosure, any one item of the wear activity information is a target information item, and the processor 12 is further configured to perform following operations:
controlling the display 14 to display detailed information of the target information item in a third display area, based on an operation instruction which is inputted by a user for the target information item, wherein the third display area partially or completely covers the first display area; or
controlling the display 14 to display a part of the wear activity information in the first display area, and to display detailed information of the target information item in the first display area, based on an operation instruction which is inputted by a user for the target information item; or
controlling the display 14 not display the wear activity information in the first display area, but to display detailed information of the target information item in the first display area, based on an operation instruction which is inputted by a user for the target information item.

In an embodiment of this disclosure, when the display mode of the bedside monitoring device 1 is the first display mode, an early-warning state evaluation of the human body is obtained, and the display 14 is controlled to display the early-warning state evaluation; wherein the early-warning state evaluation is obtained through processing the physiological parameter signal of the human body by the processor 12, or the early-warning state evaluation is the early-warning state evaluation which is generated by the mobile monitoring device 2 and received by the communication unit.

In the embodiment of this disclosure, the processor 12 is further configured to perform following operations:
when a first preset condition is satisfied, determining the display mode of the bedside monitoring device 1 to be the first display mode; wherein the first preset condition includes at least one of: a first instruction for instructing the bedside monitoring device to display in a first display mode is received, which instruction is inputted by a user on the bedside monitoring device 1; a second instruction for instructing the bedside monitoring device to display in the first display mode is received, which instruction is transmitted by the mobile monitoring device 2; an indicator, which reflects a communication state between the mobile monitoring device 2 and the bedside monitoring device 1, satisfies a first indicator condition; and an indicator, which reflects a health state of the human body, satisfies a second indicator condition;
when a second preset condition is satisfied, determining the display mode of the bedside monitoring device 1 to be the second display mode; wherein the second preset condition includes at least one of a third instruction for instructing the bedside monitoring device to display in the second display mode in received, which instruction is inputted by a user on the bedside monitoring device 1; a fourth instruction for instructing the bedside monitoring device to display in the second display mode is received, which instruction is transmitted by the mobile monitoring device 2; an indicator, which reflects a communication state between the mobile monitoring device 2 and the bedside monitoring device 1, satisfies a third indicator condition; and an indicator, which reflects a health state of the human body, satisfies a fourth indicator condition.

An embodiment of this disclosure provides a mobile monitoring device 2, wherein the monitoring mode of the mobile monitoring device 2 includes a first monitoring mode and a second monitoring mode.

Wherein a frequency of the mobile monitoring device 2 when operating in the first monitoring mode is lower than a frequency of the mobile monitoring device 2 when operating in the second monitoring mode; a frequency of the mobile monitoring device 2 when operating in the first monitoring mode being lower than a frequency of the mobile monitoring device 2 when operating in the second monitoring mode, includes at least one of following two situations: a signal acquisition frequency of the mobile monitoring device 2 when operating in the first monitoring mode is lower than a signal acquisition frequency of the mobile monitoring device 2 when operating in the second monitoring mode, and a data transmission frequency of the mobile monitoring device 2 when operating in the first monitoring mode is lower than a data transmission frequency of the mobile monitoring device 2 when operating in the second monitoring mode.

Referring to the structure of the mobile monitoring device 2 shown in FIG. 3, the mobile monitoring device 2 includes a first acquisition module 21, a second acquisition module 22, a processor 23 and a communication unit 24.

The first acquisition module 21 is configured to acquire a physiological parameter signal of a human body or process a physiological parameter signal acquired to obtain physiological parameter information of the human body.

The second acquisition module 22 is configured to a acquire non-physiological parameter signal or at least process a non-physiological parameter signal acquired to obtain wear activity information of the human body.

The communication unit 24 is configured to establish a communicative connection with a bedside monitoring device.

The processor 23 is configured to perform following operations:
when the monitoring mode of the mobile monitoring device 2 is the first monitoring mode, transmitting to the bedside monitoring device 1, through the communication unit 24, a first physiological parameter signal or first physiological parameter information, and the non-physiological parameter signal or the wear activity information, so as to enable the bedside monitoring device 1 to display the first physiological parameter information and the wear activity information; wherein the wear activity information includes at least one of: posture information, position information, movement time, and sleep time, of the human body;
when the monitoring mode of the mobile monitoring device 2 is the second monitoring mode, transmitting to the bedside monitoring device 1, through the communication unit 24, a second physiological parameter signal or second physiological parameter information, so as to enable the bedside monitoring device 1 to display the second physiological parameter information.

In an embodiment of this disclosure, when a third preset condition is satisfied, the processor 23 determines its monitoring mode as the first monitoring mode; wherein the third preset condition includes at least one of: a fifth instruction for instructing the mobile monitoring device 2 to operate in the first monitoring mode is received, which instruction is inputted by a user on the mobile monitoring device 2; a sixth instruction for instructing the mobile monitoring device 2 to operate in the first monitoring mode is received, which instruction is transmitted by the bedside monitoring device 1; an indicator, which reflects a communication state between the mobile monitoring device 2 and the bedside monitoring device 1, satisfies a first indicator condition; an indicator, which reflects a health state of the human body, satisfies a second indicator condition.

When the fourth preset condition is satisfied, the processor 23 determines its monitoring mode as the second monitoring mode; wherein the fourth preset condition includes at least one of: a seventh instruction for instructing the mobile monitoring device 2 to operate in the second monitoring mode is received, which instruction is inputted by a user on the mobile monitoring device 2; an eighth instruction for instructing the mobile monitoring device 2 to operate in the second monitoring mode is received, which instruction is transmitted by the bedside monitoring device 1; an indicator, which reflects a communication state between the mobile monitoring device 2 and the bedside monitoring device 1, satisfies a third indicator condition; an indicator, which reflects a health state of the human body, satisfies a fourth indicator condition.

An embodiment of this disclosure provides a display method for monitoring information, which is applied to a monitoring system, wherein the monitoring system includes a bedside monitoring device and a mobile monitoring device which is capable of being worn by a human body, wherein a display mode of the bedside monitoring device includes a first display mode and a second display mode, and a monitoring mode of the mobile monitoring device includes a first monitoring mode and a second monitoring mode. The main steps are as follows.

FIG. 15 is a flow chart of a display method for monitoring information provided in an embodiment of this disclosure. As shown in FIG. 15, when a display mode of a bedside monitoring device is a first display mode and a mobile monitoring device operates in a first monitoring mode, following steps are included.

S 101, the bedside monitoring device receives a physiological parameter signal of a human body acquired by the mobile monitoring device and processes the physiological parameter signal of the human body to obtain first physiological parameter information, or the bedside monitoring device receives first physiological parameter information of the human body generated by the mobile monitoring device.

S 102, the bedside monitoring device receives a non-physiological parameter signal acquired by the mobile monitoring device and at least processes the non-physiological parameter signal to obtain wear activity information, or the bedside monitoring device receives wear activity information of the human body generated by the mobile monitoring device, wherein the wear activity information includes at least one of: posture information, position information, movement time and sleep time, of the human body.

S 103, the bedside monitoring device distinguishingly displays the first physiological parameter information and the wear activity information.

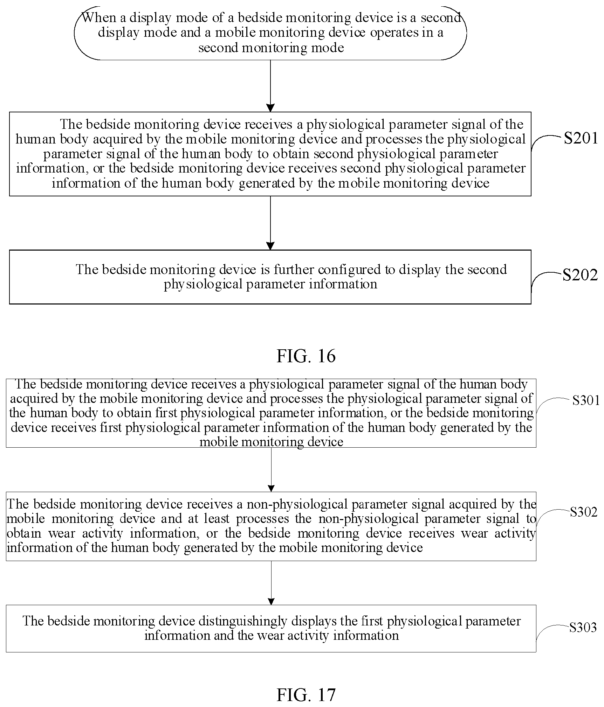

FIG. 16 is a second flow chart of a display method for monitoring information provided in an embodiment of this disclosure.

As shown in FIG. 16, when a display mode of a bedside monitoring device is a second display mode and a mobile monitoring device operates in a second monitoring mode, following steps are included.

S201, the bedside monitoring device receives a physiological parameter signal of the human body acquired by the mobile monitoring device and processes the physiological parameter signal of the human body to obtain second physiological parameter information, or the bedside monitoring device receives second physiological parameter information of the human body generated by the mobile monitoring device.

S202, the bedside monitoring device is further configured to display the second physiological parameter information.

In an embodiment of this disclosure, a frequency of the mobile monitoring device when operating in the first monitoring mode is lower than a frequency of the mobile monitoring device when operating in the second monitoring mode; a frequency of the mobile monitoring device when operating in the first monitoring mode being lower than a frequency of the mobile monitoring device when operating in the second monitoring mode, includes at least one of following two situations: a signal acquisition frequency of the mobile monitoring device when operating in the first monitoring mode is lower than a signal acquisition frequency of the mobile monitoring device when operating in the second monitoring mode, and a data transmission frequency of the mobile monitoring device when operating in the first monitoring mode is lower than a data transmission frequency of the mobile monitoring device when operating in the second monitoring mode.

An embodiment of this disclosure provides a display method for monitoring information, which is applied to a bedside monitoring device, wherein the bedside monitoring device is in communicative connection with a mobile monitoring device which is capable of being worn by a human body. FIG. 17 is a third flow chart of a display method for monitoring information provided in an embodiment of this disclosure. As shown in FIG. 17, following steps are included.

S301, the bedside monitoring device receives a physiological parameter signal of the human body acquired by the mobile monitoring device and processes the physiological parameter signal of the human body to obtain first physiological parameter information, or the bedside monitoring device receives first physiological parameter information of the human body generated by the mobile monitoring device.

S302, the bedside monitoring device receives a non-physiological parameter signal acquired by the mobile monitoring device and at least processes the non-physiological parameter signal to obtain wear activity information, or the bedside monitoring device receives wear activity information of the human body generated by the mobile monitoring device.

S303, the bedside monitoring device distinguishingly displays the first physiological parameter information and the wear activity information; wherein the wear activity information includes at least one of: posture information, position information, movement time, and sleep time, of the human body.

In an embodiment of this disclosure, font(s) of multiple first main parameter data of the first physiological parameter information is(are) larger than a font of at least one second main parameter data of the wear activity information.

In an embodiment of this disclosure, distinguishingly displaying, by the bedside monitoring device, the first physiological parameter information and the wear activity information, including: displaying the wear activity information in the first display area, and displaying the first physiological parameter information in the second display area.

In an embodiment of this disclosure, based on an operation instruction which is inputted by a user for a target information item, detailed information of the target information item is displayed in a third display area, and the third display area partially or completely covers the first display area;

Alternatively, based on an operation instruction which is inputted by a user for a target information item, part of the wear activity information is displayed in the first display area, and detailed information of the target information item is displayed in the first display area;

Alternatively, based on an operation instruction which is inputted by a user for a target information item, the wear activity information is not displayed in the first display area, but detailed information of the target information item is displayed in the first display area;

The target information item is any one item of wear activity information.

Those skilled in the art should understand that the embodiments of this disclosure may be provided as methods, systems, or computer program products. Therefore, this disclosure may take the form of a hardware embodiment, a software embodiment, or an embodiment combining software and hardware aspects. Moreover, this disclosure may take the form of a computer program product implemented on one or more computer-usable storage media (including but not limited to disk storage and optical storage, etc.) containing computer-usable program codes.

This disclosure is described with reference to flowcharts and/or block diagrams of methods, devices (systems), and computer program products according to embodiments of this disclosure. It should be understood that each process and/or block in the flowchart and/or block diagram, and a combination of processes and/or blocks in the flowchart and/or block diagram can be implemented by computer program instructions. These computer program instructions can be provided to a processor of a general-purpose computer, a special-purpose computer, an embedded processor, or other programmable signal processing device to produce a machine, so that the instructions executed by the processor of the computer or other programmable signal processing device produce a device for implementing the functions specified in one or more processes in the flowchart and/or one or more blocks in the block diagram.

These computer program instructions may also be stored in a computer-readable memory that can direct a computer or other programmable signal processing device to operate in a specific manner, so that the instructions stored in the computer-readable memory produce a product including an instruction device that implements the functions specified in one or more processes in the flowchart and/or one or more boxes in the block diagram.

These computer program instructions may also be loaded onto a computer or other programmable signal processing device so that a series of operational steps are executed on the computer or other programmable device to produce a computer-implemented process, whereby the instructions executed on the computer or other programmable device provide steps for implementing the functions specified in one or more processes in the flowchart and/or one or more boxes in the block diagram.

The above are only preferred embodiments of this disclosure and are not used to limit the protection scope of this disclosure. Any technician familiar with the technical field can easily think of changes or substitutions within the technical scope disclosed in this disclosure, which should be covered by the protection scope of this disclosure. Therefore, the protection scope of this disclosure should be based on the protection scope of the claims.

### INDUSTRIAL APPLICABILITY

Embodiments of this disclosure provide a display method for monitoring information, a monitoring system and related device, wherein the monitoring system includes: a bedside monitoring device, and a mobile monitoring device which is capable of being worn by a human body; a display mode of the bedside monitoring device includes a first display mode and a second display mode; a monitoring mode of the mobile monitoring device includes a first monitoring mode and a second monitoring mode; a frequency of the mobile monitoring device when operating in the first monitoring mode is lower than a frequency of the mobile monitoring device when operating in the second monitoring mode; wherein a frequency of the mobile monitoring device when operating in the first monitoring mode being lower than a frequency of the mobile monitoring device when operating in the second monitoring mode, includes at least one of following two situations: a signal acquisition frequency of the mobile monitoring device when operating in the first monitoring mode is lower than a signal acquisition frequency of the mobile monitoring device when operating in the second monitoring mode, and a data transmission frequency of the mobile monitoring device when operating in the first monitoring mode is lower than a data transmission frequency of the mobile monitoring device when operating in the second monitoring mode; when the display mode of the bedside monitoring device is the first display mode and the mobile monitoring device operates in the first monitoring mode, the bedside monitoring device is configured to obtain first physiological parameter information and wear activity information, and distinguishingly display the first physiological parameter information and the wear activity information; when the display mode of the bedside monitoring device is the second display mode and the mobile monitoring device operates in the second monitoring mode, the bedside monitoring device is configured to at least obtain second physiological parameter information, and display the second physiological parameter information; the first physiological parameter information and the second physiological parameter information are obtained by the bedside monitoring device through receiving and processing a physiological parameter signal of the human body acquired by the mobile monitoring device, or by the bedside monitoring device through receiving physiological parameter information of the human body generated by the mobile monitoring device; the wear activity information is obtained by the bedside monitoring device through receiving and at least processing a non-physiological parameter signal acquired by the mobile monitoring device, or by the bedside monitoring device through receiving the wear activity information of the human body generated by the mobile monitoring device; wherein the wear activity information includes at least one of: posture information, position information, movement time, and sleep time, of the human body. Technical solutions provided in embodiments of this disclosure enables a bedside monitoring device to display monitoring information in different display modes according to different monitoring modes of a mobile monitoring device, supports diverse monitoring displays, and improves a monitoring display effect.

## Claims

1. A monitoring system, **characterized in that**, comprising: a bedside monitoring device, and a mobile monitoring device which is capable of being worn by a human body;
a display mode of the bedside monitoring device comprises a first display mode and a second display mode;
a monitoring mode of the mobile monitoring device comprises a first monitoring mode and a second monitoring mode;
a frequency of the mobile monitoring device when operating in the first monitoring mode is lower than a frequency of the mobile monitoring device when operating in the second monitoring mode; wherein a frequency of the mobile monitoring device when operating in the first monitoring mode being lower than a frequency of the mobile monitoring device when operating in the second monitoring mode, comprises at least one of following two situations: a signal acquisition frequency of the mobile monitoring device when operating in the first monitoring mode is lower than a signal acquisition frequency of the mobile monitoring device when operating in the second monitoring mode, and a data transmission frequency of the mobile monitoring device when operating in the first monitoring mode is lower than a data transmission frequency of the mobile monitoring device when operating in the second monitoring mode;
when the display mode of the bedside monitoring device is the first display mode and the mobile monitoring device operates in the first monitoring mode, the bedside monitoring device is configured to obtain first physiological parameter information and wear activity information, and distinguishingly display the first physiological parameter information and the wear activity information;
when the display mode of the bedside monitoring device is the second display mode and the mobile monitoring device operates in the second monitoring mode, the bedside monitoring device is configured to at least obtain second physiological parameter information, and display the second physiological parameter information;
wherein the first physiological parameter information and the second physiological parameter information are obtained by the bedside monitoring device through receiving and processing a physiological parameter signal of the human body acquired by the mobile monitoring device, or are obtained by the bedside monitoring device through receiving physiological parameter information of the human body generated by the mobile monitoring device;
the wear activity information is obtained by the bedside monitoring device through receiving and at least processing a non-physiological parameter signal acquired by the mobile monitoring device, or is obtained by the bedside monitoring device through receiving the wear activity information of the human body generated by the mobile monitoring device;
wherein the wear activity information comprises at least one of: posture information, position information, movement time, and sleep time, of the human body.

2. The monitoring system according to claim 1, **characterized in that**, the first physiological parameter information at least comprises physiological parameter information of a numerical value type;
the second physiological parameter information comprises physiological parameter information of a numerical value type and physiological parameter information of a waveform type.

3. The monitoring system according to claim 1, **characterized in that**, distinguishingly displaying, by the bedside monitoring device, the first physiological parameter information and the wear activity information, comprises:
distinguishingly displaying the first physiological parameter information and the wear activity information in different colors and/or different marks.

4. The monitoring system according to claim 1, **characterized in that**, distinguishingly displaying, by the bedside monitoring device, the first physiological parameter information and the wear activity information, comprises:
displaying the wear activity information in a first display area, and displaying the first physiological parameter information in a second display area.

5. The monitoring system according to claim 4, **characterized in that**, the first display area and the second display area are in an up-down arrangement or a left-right arrangement.

6. The monitoring system according to claim 5, **characterized in that**, the bedside monitoring device is further configured to adjust the arrangement of the first display area and the second display area based on an adjustment instruction which is inputted by a user;
when the first display area and the second display area are in the up-down arrangement, the adjustment instruction includes an instruction for adjusting an up-down position between the first display area and the second display area, or an instruction for adjusting the first display area and the second display area to be in the left-right arrangement;
when the first display area and the second display area are in the left-right arrangement, the adjustment instruction includes an instruction for adjusting a left-right position between the first display area and the second display area, or an instruction for adjusting the first display area and the second display area to be in the up-down arrangement.

7. The monitoring system according to claim 4, **characterized in that**, any one item of the wear activity information is a target information item;
the bedside monitoring device is further configured to display detailed information of the target information item in a third display area, based on an operation instruction which is inputted by a user for the target information item, wherein the third display area partially or completely covers the first display area; or
the bedside monitoring device is further configured to display a part of the wear activity information in the first display area, and to display detailed information of the target information item in the first display area, based on an operation instruction which is inputted by a user for the target information item; or
the bedside monitoring device is further configured to not display the wear activity information in the first display area, but to display detailed information of the target information item in the first display area, based on an operation instruction which is inputted by a user for the target information item.

8. The monitoring system according to claim 4, **characterized in that**,
an area which displays the second physiological parameter information in the second display mode is a fourth display area;
respective areas occupied by the second display area in the first display mode and by the fourth display area in the second display mode are at least partially same; and/or,
in the first display mode, an area occupied by the first display area is smaller than an area occupied by the second display area; and/or,
a total area occupied by the first display area and the second display area in the first display mode is same as an area occupied by the fourth display area in the second display mode.

9. The monitoring system according to claim 1, **characterized in that**,
when the display mode of the bedside monitoring device is the first display mode and the mobile monitoring device operates in the first monitoring mode, the bedside monitoring device is further configured to obtain an early-warning state evaluation of the human body and display the early-warning state evaluation;
wherein the early-warning state evaluation is determined by the bedside monitoring device through receiving the physiological parameter signal acquired by the mobile monitoring device and at least processing the physiological parameter signal, or the early-warning state evaluation is generated by the mobile monitoring device and received by the bedside monitoring device.

10. The monitoring system according to claim 1, **characterized in that**, the posture information comprises at least one of: sleep state information, lying state information, movement state information, fall state information, and stand state information;
the bedside monitoring device is further configured to display different posture information in different icons and/or different text.

11. The monitoring system according to claim 1, **characterized in that**, the posture information comprises normal posture information and abnormal posture information;
an indication intensity which corresponds to the abnormal posture information displayed by the bedside monitoring device, is higher than an indication intensity which corresponds to the normal posture information displayed by the bedside monitoring device.

12. The monitoring system according to claim 1, **characterized in that**, the wear activity information comprises the posture information of the human body,
when the display mode of the bedside monitoring device is the first display mode and the mobile monitoring device operates in the first monitoring mode, if the posture information of the human body is fall state information, the bedside monitoring device is further configured to display first indication information which corresponds to the fall state information, wherein the first indication information comprises icon(s) and/or text, which correspond/corresponds to the fall state information, and comprises real-time position information of the human body.

13. The monitoring system according to claim 1, **characterized in that**, when a first preset condition is satisfied, the bedside monitoring device is further configured to determine its own display mode as the first display mode; wherein the first preset condition comprises at least one of:
a first instruction for instructing the bedside monitoring device to display in the first display mode is received, which instruction is inputted by a user on the bedside monitoring device;
a second instruction for instructing the bedside monitoring device to display in the first display mode is received, which instruction is transmitted by the mobile monitoring device;
an indicator, which reflects a communication state between the mobile monitoring device and the bedside monitoring device, satisfies a first indicator condition; and
an indicator, which reflects a health state of the human body, satisfies a second indicator condition.

14. The monitoring system according to claim 13, **characterized in that**, an indicator, which reflects a communication state between the mobile monitoring device and the bedside monitoring device, satisfying a first indicator condition, comprises at least one of:
a communicative connection between the mobile monitoring device and the bedside monitoring device is not a point-to-point wireless communicative connection;
when the bedside monitoring device is located inside a ward, the mobile monitoring device is located outside said ward; and
a communication distance between the mobile monitoring device and the bedside monitoring device is greater than a first preset distance threshold.

15. The monitoring system according to claim 13, **characterized in that**, an indicator, which reflects a health state of the human body, satisfying a second indicator condition, comprises at least one of:
a time length for the mobile monitoring device to monitor the human body in the second monitoring mode reaches a first time threshold;
an early-warning state evaluation of the human body is higher than a first evaluation threshold, wherein the early-warning state evaluation is determined at least based on the physiological parameter signal, or the early-warning state evaluation is transmitted by the mobile monitoring device and obtained by the mobile monitoring device at least based on the physiological parameter signal;
at least one item of the physiological parameter information of the human body has no abnormity at a current moment and/or within a preset historical time period; and
posture information of the human body is normal state information.

16. The monitoring system according to claim **1, characterized in that**, when a second preset condition is satisfied, the bedside monitoring device is further configured to determine its own display mode as the second display mode; wherein the second preset condition comprises at least one of:
a third instruction for instructing the bedside monitoring device to display in the second display mode is received, which instruction is inputted by a user on the bedside monitoring device;
a fourth instruction for instructing the bedside monitoring device to display in the second display mode, which instruction is transmitted by the mobile monitoring device;
an indicator, which reflects a communication state between the mobile monitoring device and the bedside monitoring device, satisfies a third indicator condition; and
an indicator, which reflects a health state of the human body, satisfies a fourth indicator condition.

17. The monitoring system according to claim 16, **characterized in that**, an indicator, which reflects a communication state between the mobile monitoring device and the bedside monitoring device, satisfying a third indicator condition, comprises at least one of:
a communicative connection between the mobile monitoring device and the bedside monitoring device is a point-to-point wireless communicative connection;
when the bedside monitoring device is located inside a ward, the mobile monitoring device is also located inside said ward; and
a communication distance between the mobile monitoring device and the bedside monitoring device is not greater than a second preset distance threshold.

18. The monitoring system according to claim 16, **characterized in that**, an indicator, which reflects a health state of a human body, satisfying a fourth indicator condition, comprises at least one of:
a time length for the mobile monitoring device to monitor the human body in the first monitoring mode reaches a second time threshold;
an early-warning state evaluation of the human body is lower than a second evaluation threshold, wherein the early-warning state evaluation is determined at least based on the physiological parameter signal, or the early-warning state evaluation is transmitted by the mobile monitoring device and obtained by the mobile monitoring device at least based on the physiological parameter signal;
at least one item of the physiological parameter information of the human body has an abnormity at a current moment and/or within a preset historical time period; and
posture information of the human body is abnormal state information.

19. The monitoring system according to claim 16, **characterized in that**,
when the display mode of the bedside monitoring device is the first display mode, the mobile monitoring device operates in the first monitoring mode, and the second preset condition is satisfied, the bedside monitoring device is further configured to display second indication information for switching from the first display mode to the second display mode after a preset time period.

20. The monitoring system according to claim **1, characterized in that**, the bedside monitoring device is further configured to display a countdown indication from a refreshed display for next time; wherein the countdown indication at least comprises a numerical countdown value.

21. The monitoring system according to claim 4, **characterized in that**, when the posture information and other information of the human body is displayed in the first display area, the posture information and said other information are in an up-down arrangement;
wherein said other information comprises at least one of: the position information, the movement time, the sleep time, and an early-warning state evaluation; wherein the early-warning state evaluation is determined by the bedside monitoring device through receiving the physiological parameter signal acquired by the mobile monitoring device and at least processing the physiological parameter signal, or the early-warning state evaluation is generated by the mobile monitoring device and received by the bedside monitoring device.

22. A display method for monitoring information, which method is applied to a monitoring system, **characterized in that**, the monitoring system comprises a bedside monitoring device, and a mobile monitoring device which is capable of being worn by a human body; a display mode of the bedside monitoring device comprises a first display mode and a second display mode; a monitoring mode of the mobile monitoring device comprises a first monitoring mode and a second monitoring mode;
wherein the method comprises:
when the display mode of the bedside monitoring device is the first display mode and the mobile monitoring device operates in the first monitoring mode:
receiving, by the bedside monitoring device, a physiological parameter signal of the human body acquired by the mobile monitoring device, and processing, by the bedside monitoring device, the physiological parameter signal of the human body to obtain first physiological parameter information; or receiving, by the bedside monitoring device, first physiological parameter information of the human body generated by the mobile monitoring device;
receiving, by the bedside monitoring device, a non-physiological parameter signal acquired by the mobile monitoring device, and at least processing, by the bedside monitoring device, the non-physiological parameter signal to obtain wear activity information; or receiving, by the bedside monitoring device, wear activity information of the human body generated by the mobile monitoring device, wherein the wear activity information comprises at least one of: posture information, position information, movement time, and sleep time, of the human body;
distinguishingly displaying, by the bedside monitoring device, the first physiological parameter information and the wear activity information;
when the display mode of the bedside monitoring device is the second display mode and the mobile monitoring device operates in the second monitoring mode:
receiving, by the bedside monitoring device, a physiological parameter signal of the human body acquired by the mobile monitoring device, and processing, by the bedside monitoring device, the physiological parameter signal of the human body to obtain second physiological parameter information; or receiving, by the bedside monitoring device, second physiological parameter information of the human body generated by the mobile monitoring device;
displaying, by the bedside monitoring device, the second physiological parameter information.

23. The method according to claim 22, **characterized in that**, a frequency of the mobile monitoring device when operating in the first monitoring mode is lower than a frequency of the mobile monitoring device when operating in the second monitoring mode; wherein a frequency of the mobile monitoring device when operating in the first monitoring mode being lower than a frequency of the mobile monitoring device when operating in the second monitoring mode, comprises at least one of following two situations: a signal acquisition frequency of the mobile monitoring device when operating in the first monitoring mode is lower than a signal acquisition frequency of the mobile monitoring device when operating in the second monitoring mode, and a data transmission frequency of the mobile monitoring device when operating in the first monitoring mode is lower than a data transmission frequency of the mobile monitoring device when operating in the second monitoring mode.

24. A bedside monitoring device, **characterized in that**, comprising a communication unit, a processor, a memory and a display;
the communication unit is in wireless communication with a mobile monitoring device which is capable of being worn by a human body;
the communication unit is configured to receive a physiological parameter signal of the human body acquired by the mobile monitoring device, and the processor is configured to process the physiological parameter signal of the human body, so as to obtain physiological parameter information, or the communication unit is configured to receive physiological parameter information of the human body generated by the mobile monitoring device;
the communication unit is further configured to receive a non-physiological parameter signal acquired by the mobile monitoring device, and the processor is further configured to at least process the non-physiological parameter signal, so as to obtain wear activity information, or the communication unit is further configured to receive wear activity information of the human body generated by the mobile monitoring device;
the memory is configured to store executable instructions, and the processor is further configured to execute the executable instructions, so as to enable the processor to perform following operations:
when a display mode of the bedside monitoring device is a first display mode, obtaining first physiological parameter information and the wear activity information, and controlling the display to distinguishingly display the first physiological parameter information and the wear activity information;
when a display mode of the bedside monitoring device is a second display mode, obtaining at least second physiological parameter information, and controlling the display to display the second physiological parameter information;
wherein the wear activity information comprises at least one of: posture information, position information, movement time, and sleep time, of the human body.

25. The bedside monitoring device according to claim 24, **characterized in that**, controlling the display to distinguishingly display the first physiological parameter information and the wear activity information, comprises:
controlling the display to distinguishingly display the first physiological parameter information and the wear activity information in different colors and/or different marks.

26. The bedside monitoring device according to claim 24, **characterized in that**, controlling the display to distinguishingly display the first physiological parameter information and the wear activity information, comprises:
controlling the display to display the wear activity information in a first display area and to display the first physiological parameter information in a second display area.

27. The bedside monitoring device according to claim 26, **characterized in that**, any one item of the wear activity information is a target information item; the processor is further configured to perform following operations:
controlling the display to display detailed information of the target information item in a third display area, based on an operation instruction which is inputted by a user for the target information item, wherein the third display area partially or completely covers the first display area; or
controlling the display to display a part of the wear activity information in the first display area, and to display detailed information of the target information item in the first display area, based on an operation instruction which is inputted by a user for the target information item; or
controlling the display not to display the wear activity information in the first display area, but to display detailed information of the target information item in the first display area, based on an operation instruction which is inputted by a user for the target information item.

28. The bedside monitoring device according to claim 24, **characterized in that**, the processor is further configured to perform following operations:
when the display mode of the bedside monitoring device is the first display mode, obtaining an early-warning state evaluation of the human body, and controlling the display to display the early-warning state evaluation;
wherein the early-warning state evaluation is obtained by the processor through processing the physiological parameter signal of the human body, or the early-warning state evaluation is generated by the mobile monitoring device and received by the communication unit.

29. The bedside monitoring device according to claim 24, **characterized in that**, the processor is further configured to perform following operations:
when a first preset condition is satisfied, determining the display mode of the bedside monitoring device as the first display mode; wherein the first preset condition comprises at least one of: a first instruction for instructing the bedside monitoring device to display in the first display mode is received, which instruction is inputted by a user on the bedside monitoring device; a second instruction for instructing the bedside monitoring device to display in the first display mode is received, which instruction is transmitted by the mobile monitoring device; an indicator, which reflects a communication state between the mobile monitoring device and the bedside monitoring device, satisfies a first indicator condition; and an indicator, which reflects a health state of the human body, satisfies a second indicator condition;
when a second preset condition is satisfied, determining the display mode of the bedside monitoring device as the second display mode; wherein the second preset condition comprises at least one of: a third instruction for instructing the bedside monitoring device to display in the second display mode is received, which instruction is inputted by a user on the bedside monitoring device; a fourth instruction for instructing the bedside monitoring device to display in the second display mode, which instruction is transmitted by the mobile monitoring device; an indicator, which reflects a communication state between the mobile monitoring device and the bedside monitoring device, satisfies a third indicator condition; and an indicator, which reflects a health state of the human body, satisfies a fourth indicator condition.

30. A mobile monitoring device, **characterized in that**, a monitoring mode of the mobile monitoring device comprises a first monitoring mode and a second monitoring mode;
a frequency of the mobile monitoring device when operating in the first monitoring mode is lower than a frequency of the mobile monitoring device when operating in the second monitoring mode; wherein a frequency of the mobile monitoring device when operating in the first monitoring mode being lower than a frequency of the mobile monitoring device when operating in the second monitoring mode, comprises at least one of following two situations: a signal acquisition frequency of the mobile monitoring device when operating in the first monitoring mode is lower than a signal acquisition frequency of the mobile monitoring device when operating in the second monitoring mode, and a data transmission frequency of the mobile monitoring device when operating in the first monitoring mode is lower than a data transmission frequency of the mobile monitoring device when operating in the second monitoring mode;
wherein the mobile monitoring device comprises a first acquisition module, a second acquisition module, a processor and a communication unit, wherein:
the first acquisition module is configured to acquire a physiological parameter signal of a human body, or process a physiological parameter signal acquired to obtain physiological parameter information of a human body;
the second acquisition module is configured to acquire a non-physiological parameter signal, or at least process a non-physiological parameter signal acquired to obtain wear activity information of the human body;
the communication unit is configured to establish a communicative connection with a bedside monitoring device;
the processor is configured to perform following operations:
when the monitoring mode of the mobile monitoring device is the first monitoring mode, transmitting to the bedside monitoring device, through the communication unit, a first physiological parameter signal or first physiological parameter information, and the non-physiological parameter signal or the wear activity information, so as to enable the bedside monitoring device to display the first physiological parameter information and the wear activity information; wherein the wear activity information comprises at least one of: posture information, position information, movement time, and sleep time, of the human body;
when the monitoring mode of the mobile monitoring device is the second monitoring mode, transmitting to the bedside monitoring device, through the communication unit, a second physiological parameter signal or second physiological parameter information, so as to enable the bedside monitoring device to display the second physiological parameter information.

31. The mobile monitoring device according to claim 30, **characterized in that**, when a third preset condition is satisfied, the processor is further configured to perform a following operation: determining the monitoring mode of the mobile monitoring device as the first monitoring mode;
wherein the third preset condition comprises at least one of:
a fifth instruction for instructing the mobile monitoring device to operate in the first monitoring mode is received, which instruction is inputted by a user on the mobile monitoring device;
a sixth instruction for instructing the mobile monitoring device to operate in the first monitoring mode is received, which instruction is transmitted by the bedside monitoring device;
an indicator, which reflects a communication state between the mobile monitoring device and the bedside monitoring device, satisfies a first indicator condition;
an indicator, which reflects a health state of the human body, satisfies a second indicator condition;
when a fourth preset condition is satisfied, the processor is further configured to perform a following operation: determining the monitoring mode of the mobile monitoring device as the second monitoring mode;
wherein the fourth preset condition comprises at least one of:
a seventh instruction for instructing the mobile monitoring device to operate in the second monitoring mode is received, which instruction is inputted by a user on the mobile monitoring device;
an eighth instruction for instructing the mobile monitoring device to operate in the second monitoring mode is received, which instruction is transmitted by the bedside monitoring device;
an indicator, which reflects a communication state between the mobile monitoring device and the bedside monitoring device, satisfies a third indicator condition;
an indicator, which reflects a health state of the human body, satisfies a fourth indicator condition.

32. A display method for monitoring information, which is applied to a bedside monitoring device, **characterized in that**, the bedside monitoring device is in communicative connection with a mobile monitoring device which is capable of being worn by a human body;
wherein the method comprises:
receiving, by the bedside monitoring device, a physiological parameter signal of the human body acquired by the mobile monitoring device, and processing, by the bedside monitoring device, the physiological parameter signal of the human body to obtain first physiological parameter information; or receiving, by the bedside monitoring device, first physiological parameter information of the human body generated by the mobile monitoring device;
receiving, by the bedside monitoring device, a non-physiological parameter signal acquired by the mobile monitoring device, and at least processing, by the bedside monitoring device, the non-physiological parameter signal to obtain wear activity information; or receiving, by the bedside monitoring device, wear activity information of the human body generated by the mobile monitoring device;
distinguishingly displaying, by the bedside monitoring device, the first physiological parameter information and the wear activity information;
wherein the wear activity information comprises at least one of: posture information, position information, movement time, and sleep time, of the human body.

33. The method according to claim 32, **characterized in that**, font(s) of multiple first main parameter data of the first physiological parameter information is(are) larger than a font of at least one second main parameter data of the wear activity information.

34. The method according to claim 32, **characterized in that**, distinguishingly displaying, by the bedside monitoring device, the first physiological parameter information and the wear activity information, comprises:
displaying the wear activity information in a first display area, and displaying the first physiological parameter information in a second display area.

35. The method according to claim 34, **characterized in that**, further comprising:
displaying detailed information of a target information item in a third display area, based on an operation instruction which is inputted by a user for the target information item, wherein the third display area partially or completely covers the first display area; or
displaying a part of the wear activity information in the first display area, and detailed information of a target information item in the first display area, based on an operation instruction which is inputted by a user for the target information item; or
not displaying the wear activity information in the first display area, but displaying detailed information of a target information item in the first display area, based on an operation instruction which is inputted by a user for the target information item.
wherein the target information item is any one item of the wear activity information.

36. A bedside monitoring device, **characterized in that**, comprising a communication unit, a processor, a memory and a display;
the communication unit is in wireless communication with a mobile monitoring device which is capable of being worn by a human body;
the communication unit is configured to receive a physiological parameter signal of the human body acquired by the mobile monitoring device, and the processor is configured to process the physiological parameter signal of the human body, so as to obtain physiological parameter information, or the communication unit is configured to receive physiological parameter information of the human body generated by the mobile monitoring device;
the communication unit is further configured to receive a non-physiological parameter signal acquired by the mobile monitoring device, and the processor is further configured to at least process the non-physiological parameter signal, so as to obtain wear activity information, or the communication unit is further configured to receive wear activity information of the human body generated by the mobile monitoring device;
the memory is configured to store executable instructions, and the processor is further configured to execute the executable instructions, so as to enable the processor to perform following operations:
acquiring first physiological parameter information and the wear activity information, and controlling the display to distinguishingly display the first physiological parameter information and the wear activity information;
wherein the wear activity information comprises at least one of: posture information, position information, movement time, and sleep time, of the human body.

37. The bedside monitoring device according to claim 36, **characterized in that**, font(s) of multiple first main parameter data of the first physiological parameter information is(are) larger than a font of at least one second main parameter data of the wear activity information.

38. The bedside monitoring device according to claim 36, **characterized in that**, controlling the display to distinguishingly display the first physiological parameter information and the wear activity information, comprises:
controlling the display to distinguishingly display the first physiological parameter information and the wear activity information in different colors and/or different marks.

39. The bedside monitoring device according to claim 36, **characterized in that**, controlling the display to distinguishingly display the first physiological parameter information and the wear activity information, comprises:
controlling the display to display the wear activity information in a first display area and to display the first physiological parameter information in a second display area.

40. The bedside monitoring device according to claim 39, **characterized in that**, the processor is further configured to perform following operations:
controlling the display to display detailed information of a target information item in a third display area, based on an operation instruction which is inputted by a user for the target information item, wherein the third display area partially or completely covers the first display area; or
controlling the display to display a part of the wear activity information in the first display area, and detailed information of a target information item in the first display area, based on an operation instruction which is inputted by a user for the target information item; or
controlling the display not to display the wear activity information in the first display area, but to display detailed information of a target information item in the first display area, based on an operation instruction which is inputted by a user for the target information item;
wherein the target information item is any one item of the wear activity information.

41. The bedside monitoring device according to claim 37, **characterized in that**, the first main parameter data is data of a numerical value type;
the second display area comprises multiple sub-display areas, at least some of the multiple sub-display areas have a same area, one sub-display area is configured to display one first main parameter data; wherein an area, which is occupied by the first main parameter data displayed in each sub-display area, exceeds a preset threshold of an overall area of said sub-display area.

42. The bedside monitoring device according to claim 36, **characterized in that**, the first physiological parameter information comprises first auxiliary parameter data of a numerical value type, and/or data of at least one waveform type.

43. A mobile monitoring device, **characterized in that**, comprising a first acquisition module, a second acquisition module, a processor and a communication unit, wherein:
the first acquisition module is configured to acquire a physiological parameter signal of a human body, or process a physiological parameter signal acquired to obtain physiological parameter information of the human body;
the second acquisition module is configured to acquire a non-physiological parameter signal, or at least process a non-physiological parameter signal acquired to obtain wear activity information of the human body;
the communication unit is configured to establish a communicative connection with a bedside monitoring device;
the processor is configured to perform following operations:
transmitting to the bedside monitoring device, through the communication unit, a first physiological parameter signal or first physiological parameter information; and transmitting to the bedside monitoring device, through the communication unit, the non-physiological parameter signal or the wear activity information, so as to enable the bedside monitoring device to display the first physiological parameter information and the wear activity information;
wherein the wear activity information comprises at least one of: posture information, position information, movement time, and sleep time, of the human body.

44. A monitoring system, **characterized in that**, comprising: a bedside monitoring device, and a mobile monitoring device which is capable of being worn by a human body;
wherein a communicative connection is established between the bedside monitoring device and the mobile monitoring device;
wherein, the bedside monitoring device is the bedside monitoring device according to any one of claims 36~42.
